# EUROPEAN PATENT APPLICATION

(11) **EP 4 563 705 A1**
(43) Date of publication of application: **04.06.2025**
(21) Application number: 23213203.5
(22) Date of filing: 30.11.2023
(51) Int. Cl.: C12P 7/02, C07C 35/08, C12N 9/90

(54) **BIOCATALYTIC STEREOCONTROLLED PRODUCTION OF 1-(R)-CIS-PMD**

(71) Applicant: Universität Stuttgart, 70174 Stuttgart (DE)
(72) Inventor: HAUER, Bernhard, 67136 Fußgönheim (DE); SCHNEIDER, Andreas, 70771 Leinfelden-Echterdingen (DE)
(74) Representative: Schrell, Andreas

(57) **Abstract**

The present invention relates to a process for preparing a composition comprising 1-(R)-cis-PMD (para-menthane-3,8-diol), proteins, in particular enzymes, for use in such a process and a composition obtainable by the process.

## Description

The present invention relates to a process for preparing a composition comprising 1-(R)-cis-PMD (para-menthane-3,8-diol), proteins, in particular enzymes, for use in such a process and a composition obtainable by the process.

Mosquito-borne diseases such as malaria, dengue, chikungya, yellow fever and the Zika virus threaten millions of people every year, with global climate change exacerbating this threat even further. (World Health Organization, 2020; Beermann *et al.,* 2023) In this regard, the use of mosquito repellents, shown in Scheme 1, as *N,N-*diethyl-meta-toluamide 1 (DEET), icaridin 2, ethyl 3-(acetyl(butyl)amino]-propanoate 3 (IR3535), and *para*-menthane-3,8-diol 4 (PMD) are a promising strategy to fight against this scourge.

While DEET has long been recognized as the golden standard mosquito repellent, recent studies suggest adverse effects, e.g. neurotoxicity, on human health of this and other synthetic repellents (Swale *et al.,* 2014; Abd-Ella *et al.,* 2015). On the other hand, it has been shown that the naturally sourced PMD displays a similar (Drapeau *et al.,* 2011) and in some studies even better (Carroll and Loye, 2006) repellent activity to that of DEET, while also having low toxicity and minor adverse effects, such as skin irritation. The natural resource of PMD is *Eucalyptus citriodora,* where it constitutes a minor amount of the leaves extract. Hence synthetic processes for PMD production have been developed to satisfy the market demand.

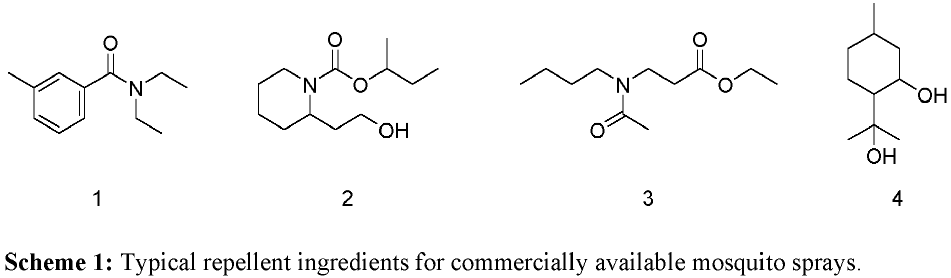

As shown in Scheme 2, PMD 4 is typically generated by electrophilic activation, e.g. a Lewis- or Brønsted acid, of the carbonyl moiety and subsequent cyclohydration (in the following also termed Path B), of citronellal 5, which was already found in 1897 by Barbier and Leser (Barbier and Leser, 1897). According to this reaction, a number of procedures emerged, e.g. using sulfuric acid in the patent U.S. Pat. No. 5,959,161 or Lewis acid catalysts, which are corrosive, require toxic solvents, and generate much waste during purification steps (Kočovský *et al.,* 1999). Consequently, ecologically friendly processes have been developed employing, for instance. citric acid (EP2862442), carbon dioxide in water (Cheng *et al.,* 2009), lignin-derived carbon acids (Kurnia *et al.,* 2020) or ammonium salts (Stanovych *et al.,* 2023) as catalysts to promote citronellal 5 cyclization.

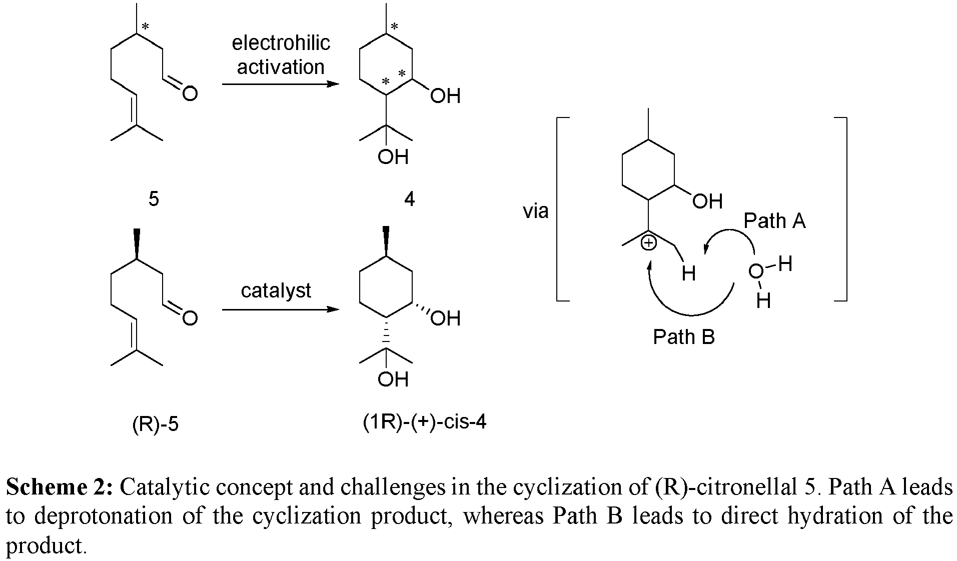

A challenge in the aforementioned reaction is not only to control cyclohydration (cyclization and hydration), Path B, over cyclization (in the following also termed Path A) but also to selectively generate the two emerging stereocenters at the cyclohexane ring.

The importance of stereoselectivity has recently been elucidated in a study using Aedes albopictus, which are responsible for chikungunya, dengue, lymphatic filariasis, Rift Valley fever, yellow fever, and Zika transmission (Borrego *et al.,* 2021). Here the authors highlight a "significantly higher efficacy and duration of protection" of the (1R)-(+)-*cis*-PMD 4 isomer (in the following also termed cis-4 or 1-(R)-cis-PMD), against the other PMD isomers. Thus, higher ratios of cis-4 in the final product could reduce the required overall amount of PMD in the mosquito spray and save costs and resources. However, the best reported ratio of 88:12 (cis:trans) needs cryogenic conditions (-78° C), a pH of 1, and 30 times as much acid as the substrate (R)-citronellal 5 (in the following also termed (R)-5) (Borrego *et al.,* 2021). Regarding stereocontrol, the Hauer group demonstrated in 2017 the highly stereoselective cyclization, Path A, of (R)-5 to (+)-neo-isopulegol, which possesses the desired stereochemistry, employing a mutant of the squalene-hopene cyclase (SHC) from Alicyclobacillus acidocaldarius (Aac) that was expressed in and isolated from E. coli, as well as purified via Ion Exchange chromatography (Bastian *et al.,* 2017). In this study no PMD 4 was described as an occurring product.

The technical problem underlying the present invention therefore is to provide means and methods for the preparation of compositions comprising cis-4, which overcome the above identified problems, in particular provide (1R)-(+)-cis-PMD in a highly stereroselective way, in particular promote the cyclohydration, of (R)-citronellal in a highly stereoselective, ecologically friendly, that means waste-reducing, non-toxic and non-corrosive, preferably in high yields and in a cost-efficient manner.

The technical problems underlying the present invention are in particular solved by the teaching of the independent and dependent claims and the accompanying description.

The technical problem underlying the present invention is in particular solved by the provision of a process for preparing a composition comprising 1-(R)-cis-PMD, comprising the following steps:
x) providing (R)-citronellal, at least one reaction medium and at least one biocatalyst comprising or consisting of a protein with the enzymatic activity of a Squalene-hopene-cyclase (SHC), which protein comprises
   - a mutated amino acid sequence relative to the wild type SHC of Alicyclobacillus acidocaldarius (wild type AacSHC) of SEQ ID No. 1, wherein the mutated amino acid sequence comprises the amino acid sequence of SEQ ID No. 1 and wherein in SEQ ID No. 1 at least the F at position 365 is replaced by an amino acid selected from the group consisting of A, C, H, I, M, P, S, T and Y or a functional equivalent of the mutated amino acid sequence or
   - a mutated amino acid sequence relative to a wild type SHC (WT-SHC), wherein the mutated amino acid sequence comprises the amino acid sequence of the WT-SHC and wherein in the WT-SHC at least the F at a position in the amino acid sequence of the WT-SHC corresponding to F in the wild type AacSHC of SEQ ID No1 at position 365 is replaced by an amino acid selected from the group consisting of A, C, H, I, M, P, S, T and Y or a functional equivalent of the mutated amino acid sequence and
y) mixing the (R)-citronellal with the reaction medium and the biocatalyst so as to obtain a concentration, in particular an initial concentration, of at least 4 mM (R)-citronellal in the reaction medium and reacting the (R)-citronellal with the biocatalyst under reaction conditions so as to convert the (R)-citronellal to a composition comprising 1-(R)-cis-PMD.

Thus, the present process uses according to process step x) as starting material for the preparation of cis-4 (R)-citronellal, which may be present in pure form or in form of a composition comprising (R)-citronellal. Further, at least one reaction medium, in particular one reaction medium and at least one, in particular one biocatalyst is provided. Advantageously, the biocatalyst used according to the present invention comprises a protein with the enzymatic activity of a Squalene-hopene-cyclase (SHC), and thus an enzyme, which is capable to catalyse the cyclohydration of (R)-citronellal to 1-(R)-cis-PMD in a very favorable way. In the subsequent process step b) the at least three components are brought into contact with each other, in particular are mixed which each other. In particular, the invention requires to mix these components so as to reach an initial, that means starting, concentration of the (R)-citronellal of at least 4 mM in the reaction medium. Thus, the corresponding amounts of reaction medium and (R)-citronellal have to be provided in step a) Subsequently to mixing, the conversion of (R)-citronellal to 1-(R)-cis-PMD, which is according to the present invention a cyclohydration, takes place under suitable reaction conditions. Thus, in step y) the conversion leads to the preparation of a composition, which comprises 1-(R)-cis-PMD - due to the high stereoselectivity and good yield of the present process - in a high concentration and with rarely any by-products.

The presently provided process is advantageous since it provides 1-(R)-cis-PMD in a highly stereoselective way, in particular promotes the cyclohydration of (R-)-citronellal in a highly stereoselective, ecologically friendly, that means waste-reducing, non-toxic and non-corrosive way by using mutant SHCs, preferably in high yields and in a cost-efficient manner. Further, the present process enables a very favorable, cost-effective preparation of 1-(R)-cis-PMD in a high yield and with a high specifity.

The protein with the enzymatic activity of a Squalene-hopene-cyclase (SHC) used and provided according to the present invention comprises, in particular consists, of a mutated amino acid sequence, which mutated amino acid sequence is mutated relative, herein also termed "in comparison", to the amino acid sequence of a wild-type SHC.

The "protein with the enzymatic activity of a Squalene-hopene-cyclase (SHC) comprising a mutated amino acid sequence" of the present invention is hereinafter also termed a "mutant SHC".

The mutated amino acid sequence of the mutant SHC used in the present process comprises, in particular consists, of the wild-type amino acid sequence of a SHC except for the presence of at least one, in particular one, two or three, amino acid mutation in the mutant SHC, which mutation is the replacement (hereinafter also termed "exchange") of a particular amino acid in the amino acid sequence of the WT-SHC, in particular is the replacement of the F at position 365 (F365) of SEQ ID No. 1 of the wild type AacSHC by an amino acid selected from the group consisting of A, C, H, I, M, P, S, T and Y or -- with respect to amino acid sequences of other WT-SHCs - is the replacement of the F at a position corresponding to F365 of SEQ ID No. 1 in the WT-SHC by an amino acid selected from the group consisting of A, C, H, I, M, P, S, T and Y.

Thus, in a preferred embodiment the mutated amino acid sequence comprises at least a mutation selected from the group consisting of F365A, F365C, F365H, F365I, F365M, F365P, F365S, F365T and F365Y.

Accordingly, the mutant SHC is characterized by comprising, in particular consisting of, an amino acid sequence, which is identical to the wild-type amino acid sequence of a Squalene-hopene-cyclase from natural sources, for instance plant or microbial sources, most preferred from microbial, in particular bacterial source, except for at least one, in particular one, amino acid exchange in this wild-type amino acid sequence rendering the amino acid sequence a mutated amino acid sequence. The mutated amino acid sequence of the protein used according to the invention comprising the at least one, in particular one, two or three, amino acid exchange compared to the amino acid sequence of the wild type SHC enables a highly improved conversion of (R)-citronellal to 1-(R)-cis-PMD, in particular a composition comprising 1-(R)-cis-PMD (-), preferably consisting of 1-(R)-cis-PMD.

In a preferred embodiment, the wild type AacSHC or the WT-SHC comprises 1, 2 or 3 mutations, that means amino acid exchanges.

Surprisingly, the invention shows that that specific mutants of SHC's are capable to efficiently convert (R)-citronellal to 1-(R)-cis-PMD with a high stereoselectivity, which phenomenon may, without being limited by theory, be due to specific changes in the accessibility and size of the substrate- and water-binding pockets and accompanying changes in the orientation and/or position of the substrate and/or water molecule in said pockets. Without being bound by theory, it appears as if the specific identity of the amino acids being capable to replace the F at position 365 and thereby allowing the stereoselective preparation of 1-(R)-cis-PMD from (R)-citronellal is crucial therefor. Without being bound by theory, it appears as if the size and limited, not too strong polarity of the amino acid replacing the F at position 365 of the present invention possibly modifies, in particular increases, the size of the substrate binding pocket, while ensuring hydrophobicity for binding of (R)-citronellal.

According to a preferred embodiment of the present invention, the mutant SHC is characterized by comprising, in particular consisting of, a mutated amino acid sequence which is identical to the amino acid sequence of a wild type SHC of Alicyclobacillus acidocaldarius (wild type AacSHC) of SEQ ID No. 1 except for at least the exchange, in particular the exchange, of F365 in the amino acid sequence of the wild type of Alicyclobacillus acidocaldarius (wild type AacSHC of SEQ ID No. 1) by an amino acid selected from the group consisting of A, C, H, I, M, P, S, T and Y.

According to a preferred embodiment of the present invention, the protein with the enzymatic activity of a Squalene-hopene-cyclase (SHC) is characterized by comprising, in particular consisting of, a mutated amino acid sequence which is identical to the amino acid sequence of a wild type SHC (WT-SHC) except for at least the exchange of the F at a position in the amino acid sequence of the WT-SHC corresponding to the F365 in the wild type AacSHC of Alicyclobacillus acidocaldarius of SEQ ID No. 1 by an amino acid selected from the group consisting of A, C, H, I, M, P, S, T and Y.

According to a preferred embodiment of the present invention, the mutant SHC is characterized by comprising, in particular consisting of, a mutated amino acid sequence which is identical to the amino acid sequence of a WT-SHC of any one of SEQ ID No. 2 to 5 except for at least the exchange, in particular the exchange, of F365 at a position corresponding to F365 of SEQ ID No. 1 in the amino acid sequence of the WT-SHC according to any one of SEQ ID No. 2 to 5 by an amino acid selected from the group consisting of A, C, H, I, M, P, S, T and Y.

In a preferred embodiment, the F at position 365 in the wild type AacSHC of SEQ ID No. 1 or the F at a position in the amino acid sequence of the WT-SHC corresponding to F in the wild type AacSHC of SEQ ID No. 1 at position 365 is replaced by an amino acid selected from the group consisting of A, P, S, T and Y, preferably by A.

In a preferred embodiment, the present invention relates to a functional equivalent of the mutated amino acid sequence relative to the wild type AacSHC of SEQ ID No. 1, that means relates to a functional equivalent comprising a derivatized mutated amino acid sequence, which derivatized amino acid sequence has an amino acid sequence identity from 38,0 to 99,9 %, preferably 45,0 to 99,9 %, preferably from 50,0 to 99,9 %, preferably from 60,0 to 99,9 %, preferably from 70 to 99,9 %, preferably from 80 to 99,9 %, preferably from 90,0 to 99,9 %, preferably from 95,0 to 99,9 %, preferably from 98,0 to 99,9 % to the amino acid sequence of the mutated amino acid sequence and which is able to convert (R)-citronellal to 1-(R)-cis-PMD, in particular a composition comprising 1-(R)-cis-PMD, preferably consisting of 1-(R)-cis-PMD with a stereoselectivity at least as high as the mutant SHC itself.

In a preferred embodiment, the present invention relates to a functional equivalent of the mutated amino acid sequence relative to the WT-SHC, that means relates to a functional equivalent comprising a derivatized mutated amino acid sequence, which derivatized amino acid sequence has an amino acid sequence identity from 38,0 to 99,9 %, preferably 45,0 to 99,9 %, preferably from 50,0 to 99,9 %, preferably from 60,0 to 99,9 %, preferably from 70 to 99,9 %, preferably from 80 to 99,9 %, preferably from 90,0 to 99,9 %, preferably from 95,0 to 99,9 %, preferably from 98,0 to 99,9 % to the amino acid sequence of the mutated amino acid and which is able to convert (R)-citronellal to 1-(R)-cis-PMD, in particular a composition comprising 1-(R)-cis-PMD, preferably consisting of 1-(R)-cis-PMD with a stereoselectivity at least as high as the mutant SHC itself with a stereoselectivity at least as high as the mutant SHC itself.

The protein in form of the functional equivalent used according to the invention is, thus, characterized by its enzymatic activity, in particular by its ability to enzymatically catalyze, in particular in a liquid reaction medium, at least the cyclohydration of (R)-citronellal to 1-(R)-cis-PMD, in particular a composition comprising 1-(R)-cis-PMD, preferably consisting of 1-(R)-cis-PMD with a stereoselectivity at least as high as the mutant SHC itself.

The protein used according to the invention catalyzes in one preferred embodiment the conversion of (R)-citronellal to a composition comprising 1-(R)-cis-PMD, which composition comprises 1-(R)-cis-PMD and further components, in particular by-products, which by-product is in particular the 1-(R)-trans-PMD enantiomer, one of a combination of the 4 stereoisomeric isopulegols or any combination thereof. Such a composition preferably can be an enantiomeric enriched and/or diastereomeric enriched composition of 1-(R)-cis-PMD.

In a further preferred embodiment, the protein used according to the invention catalyzes the conversion of (R)-citronellal to a composition consisting of 1-(R)-cis-PMD, which is essentially pure, preferably pure, 1-(R)-cis-PMD, in particular an enantiomeric pure and diastereomeric pure 1-(R)-cis-PMD.

According to the invention, the protein used shows a very high stereoselectivity, product selectivity, relative conversion or a combination of two or three of them, in the cyclohydration reaction of (R)-citronellal to a composition comprising 1-(R)-cis-PMD, preferably consisting of 1-(R)-cis-PMD.

According to the invention, the protein used shows a stereoselectivity in terms of a diastereomeric excess of at least 40, at least 50, at least 60, at least 70, at least 80 or preferably at least 90 %, (each in de (%)), in the cyclohydration reaction of (R)-citronellal to 1-(R)-cis-PMD.

According to the invention, the protein used shows a product selectivity of at least 10, at least 20, at least 30, at least 40, at least 50 at least 60, at least 70 or preferably at least 80 % in the cyclohydration reaction of (R)-citronellal to 1-(R)-cis-PMD (based on the amount of cis-4 on all products).

According to the invention, the protein used shows a relative conversion of at least 10, at least 20, at least 30, least 40, at least 50, at least 60, at least 70, at least 80 or preferably at least 90 %, (each in % of substrate), in the cyclohydration reaction of (R)-citronellal to 1-(R)-cis-PMD.

The technical problem underlying the present invention is in particular also solved by the provision of proteins with the enzymatic activity of a Squalene-hopene-cyclase (SHC), which proteins can advantageously and in a preferred embodiment be used in a process of the present invention and which protein is comprising
a) a mutated amino acid sequence relative to the wild type SHC of Alicyclobacillus acidocaldarius (wild type AacSHC) of SEQ ID No. 1, wherein the mutated amino acid sequence comprises the amino acid sequence of SEQ ID No. 1 and wherein in SEQ ID No. 1 at least the F at position 365 and the L at position 607 are mutated by an amino acid replacement or a functional equivalent of the mutated amino acid sequence or
b) a mutated amino acid sequence relative to a wild type SHC (WT-SHC), wherein the mutated amino acid sequence comprises the amino acid sequence of the WT-SHC and wherein in the WT-SHC at least the F at a position in the amino acid sequence of the WT-SHC corresponding to F365 in the wild type AacSHC of SEQ ID No. 1 and the L at a position in the amino acid sequence of the WT-SHC corresponding to L607 in the wild type AacSHC of SEQ ID No. 1 are mutated by an amino acid replacement or a functional equivalent of the mutated amino acid sequence.

In a preferred embodiment, the wild type AacSHC or the WT-SHC comprises 1, 2 or 3 mutations, that means amino acid exchanges.

The presently provided protein with the enzymatic activity of a Squalene-hopene-cyclase (SHC) is characterized by a particular high stereoselectivity for the conversion of (R)-citronellal to 1-(R)-cis-PMD at high conversion rates.

Thus, according to the invention, a protein is provided with the enzymatic activity of a SHC, which shows a very high stereoselectivity, product selectivity, relative conversion or a combination of two or three of them, in the cyclohydration reaction of (R)-citronellal to a composition comprising 1-(R)-cis-PMD, preferably consisting of 1-(R)-cis-PMD.

The mutated amino acid sequence of the mutant SHC or functional equivalent of the provided protein shows a stereoselectivity in terms of a diastereomeric excess of at least at least 70, at least 80 or preferably at least 90 % (each in de (%) and based on 1-(R) cis and trans-PMD), in the cyclohydration reaction of (R)-citronellal to 1-(R)-cis-PMD.

The mutated amino acid sequence of the mutant SHC or functional equivalent of the provided protein shows a stereoselectivity in terms of an enantiomeric excess of at least 90, or preferably at least 95 %, preferably at least 99% (each in ee (%) and based on 1-(R) cis and 1-(S) cis-PMD).

The mutated amino acid sequence of the mutant SHC or functional equivalent shows a product selectivity of at least 60, at least 70 or preferably at least 80 % in the cyclohydration reaction of (R)-citronellal to 1-(R)-cis-PMD (each in ps (%) and based on all products).

The mutated amino acid sequence of the mutant SHC or functional equivalent shows a relative conversion of at least 20, at least 30, least 40, at least 50, at least 60, at least 70, at least 80 or preferably at least 90 %, (each in rc (%) of products and substrate), in the cyclohydration reaction of (R)-citronellal to 1-(R)-cis-PMD.

In a preferred embodiment the invention provides a protein of the present invention or uses a protein in a present process, wherein a) in SEQ ID No. 1 at least the F at position 365 is mutated by replacement with an amino acid selected from the group consisting of A, C, H, I, M, P, S, T and Y, or wherein b) at least the amino acid, in particular F, at a position in the amino acid sequence of the WT-SHC corresponding to F365 in the wild type AacSHC of SEQ ID No. 1 is replaced by an amino acid selected from the group consisting of A, C, H, I, M, P, S, T and Y.

In a preferred embodiment the invention provides a protein of the present invention or uses a protein in a present process, wherein a) in SEQ ID No. 1 at least the F at position 365 is mutated by replacement with an amino acid selected from the group consisting of A, P, S, T and Y, preferably by A, or wherein b) at least the amino acid, in particular F, at a position in the amino acid sequence of the WT-SHC corresponding to F365 in the wild type AacSHC of SEQ ID No. 1 is replaced by an amino acid selected from the group consisting of A, P, S, T and Y, preferably by A.

In a preferred embodiment the invention provides a protein of the present invention or uses a protein in a present process, wherein a) in SEQ ID No. 1 at least the L at position 607 is mutated by replacement with an amino acid selected from the group consisting of A, D, G, M and N, preferably by G or M, or b) at least the amino acid, in particular L, at a position in the amino acid sequence of the WT-SHC corresponding to L607 in the wild type AacSHC of SEQ ID No. 1 is replaced by an amino acid selected from the group consisting of A, D, G, M and N, preferably by G or M.

Thus, in a preferred embodiment the mutated amino acid sequence comprises at least a mutation selected from the group consisting of F365A, F365C, F365H, F365I, F365M, F365P, F365S, F365T and F365Y and a mutation selected from the group consisting of L607A, L607D, L607G, L607M and L607N.

Thus, in a preferred embodiment the mutated amino acid sequence comprises at least a mutation selected from the group consisting of F365A, F365P, F365S, F365T and F365Y and a mutation selected from the group consisting of L607A, L607D, L607G, L607M and L607N.

In a preferred embodiment the invention provides a protein of the present invention or uses a protein in a present process, wherein further, that means in addition to the mutations at positions F365 and L607 a) in SEQ ID No. 1 at least the G at position 600 is mutated, in particular by replacement with F, L, M or V or b) at least the G at a position in the amino acid sequence of the WT-SHC corresponding to G600 in the wild type AacSHC of SEQ ID No. 1 is mutated, in particular by replacement with F, L, M or V, preferably F.

Thus, in a preferred embodiment the mutated amino acid sequence comprises in addition to the mutations at positions F365 and L607 at least the mutations G600F, G600L, G600M or G600V.

In a preferred embodiment the invention provides a protein of the present invention or uses a protein in a present process, wherein the mutated amino acid sequence relative to the wild type SHC of Alicyclobacillus acidocaldarius comprises at least mutations selected from the group consisting of F365A/L607G, F365A/L697M, F365A/G600F/L607M and F365A/G600F/L607G or the mutated amino acid sequence relative to a wild type SHC comprises mutations selected from the group consisting of the corresponding mutations of F365A/L607G, F365A/L697M, F365A/G600F/L607M and F365A/G600F/L607G in the wild type SHC.

In a preferred embodiment the invention provides a protein of the present invention or uses a protein in a present process,
wherein the mutated amino acid sequence relative to the wild type AacSHC of SEQ ID No. 1 is the amino acid sequence of any one of SEQ ID Nos 6 to 9, or
wherein the WT-SHC is selected from the group consisting of WT-SHC of Streptomyces coelicolor (ScoSHC, SEQ ID No. 2), Zymomonas mobilis (ZmoSHC1, SEQ ID No. 3, ZmoSHC2, SEQ ID No. 4) and Thermosynechococcus elongatus (TelSHC, SEQ ID No. 5) or
wherein the mutated amino acid sequence relative to a WT-SHC Streptomyces coelicolor, in particular of SEQ ID No 2, is the amino acid sequence of SEQ ID 10.

### Preferred reaction conditions of the present process

In a particularly preferred embodiment, the reaction medium in step y) is water, at least one buffer, in particular at least one aqueous buffer or a buffer system of two of them.

In a particularly preferred embodiment, the reaction medium is free of a detergent, in particular free of Triton X-100.

In a particularly preferred embodiment, the reaction medium in step y) comprises water, preferably is water, preferably demineralized water, preferably distilled water.

In a preferred embodiment, the reaction medium in step y) comprises, preferably consists of, a buffer, in particular an aqueous buffer, in particular selected from the group of buffering agents consisting of phosphate buffer, in particular potassium phosphate buffer, in particular 10x phosphate buffer, a potassium buffer, in particular MES/potassium buffer, in particular MES/KOH buffer, TRIS-Maleate buffer, citrate buffer and acetate buffer, in particular citrate buffer.

In a preferred embodiment, the reaction temperature in step y) is 20 °C to 50 °C, in particular 22 °C to 42 °C, in particular 25 °C to 40 °C, in particular 28 °C to 32 °C, in particular 30 °C.

In a preferred embodiment, the present process for preparing a composition comprising 1-(R)-cis-PMD, uses a reaction medium, which has a pH of at least 5,0, most preferably at least 6,1, most preferably is from 6,1 to 8,5, most preferably from 6,4 to 8,0 (each measured at 20 °C).

In a preferred embodiment, the present process for preparing a composition comprising 1-(R)-cis-PMD uses amounts of reaction medium and (R)-citronellal in steps x) and y) so as to obtain a concentration of at least 5, at least 7, at least 9 or preferably of at least 10 mM (R)-citronellal in the reaction medium.

In a preferred embodiment, the present process for preparing a composition comprising 1-(R)-cis-PMD, uses amounts of reaction medium and (R)-citronellal in steps x) and y) so as to obtain a concentration of 5 to 30, in particular 7 to 25, preferably 10 to 20 mM (R)-citronellal in the reaction medium.

In a preferred embodiment, the present process is further comprising subsequent to step y) a step x) of isolating 1-(R)-cis-PMD from the composition comprising 1-(R)-cis-PMD obtained in step y). The isolation may be conducted by chromatography or in situ product crystallization.

According to a preferred embodiment of the present process in step x) an encapsulating agent, in particular cyclodextrin, in particular γ-cyclodextrin, is provided and mixed in step y) with the reaction medium and the biocatalyst.

It was surprisingly found, that the encapsulating agent, in particular cyclodextrin, prevents the undesired formation acetals during the reaction, in particular cylcohydration, Further, the presence of cyclodextrin in a composition comprising 1-(R)-cis-PMD, in particular obtainable according to the invention, prolongs the insect repellent effect of 1-(R)-cis-PNM.

According to a preferred embodiment of the present process, the encapsulating agent, in particular cyclodextrin, in particular γ-cyclodextrin is provided and mixed in equimolar concentration to the (R)-citronellal in the reaction medium.

According to a preferred embodiment of the present process, the encapsulating agent, in particular cyclodextrin, in particular γ-cyclodextrin, is contained in the reaction medium in a concentration of at least 2, at least 5, at least 7 or at least 9 or preferably of at least 10 mM.

According to a preferred embodiment of the present process, the encapsulating agent, in particular cyclodextrin, in particular γ-cyclodextrin, is contained in the reaction medium in a concentration of 2 to 40, of 5 to 30, in particular of 7 to 25, preferably of 10 to 20 mM.

In a preferred embodiment, the biocatalyst is used in the reaction medium in step y) in a quantity that in the reaction medium a concentration from 0,001 to 100 µmol/l, preferably 0,01 to 90 µmol/l, preferably 0,1 to 80 µmol/l, preferably 1 to 75 µmol/l, preferably 10 to 70 µmol/l, preferably in particular 40 to 60 µmol/l of the protein in relation to the reaction medium is present.

In a preferred embodiment, the biocatalyst is used in the reaction medium in step y) in a quantity that in the reaction medium a concentration from 0,001 to 100 µmol/l, preferably 0,01 to 90 µmol/l, preferably 0,1 to 80 µmol/l, preferably 1 to 75 µmol/l, preferably 10 to 70 µmol/l, preferably in particular 40 to 60 µmol/l of the protein, in relation to the amount of water or buffer in the reaction medium is present.

In a preferred embodiment, the biocatalyst, in particular host cell, is used in the reaction medium in step c)) at a cell concentration of 0,1 to 100 mg/ml, in particular 10 to 80 mg/ml, in particular 30 to 60 mg/ml, in particular 50 mg/ml, in relation to the reaction medium (wet weight to total volume of reaction medium).

In a preferred embodiment, the biocatalyst, in particular host cell, is used in the reaction medium in step y) at a cell concentration of 0,1 to 100 mg/ml, in particular 10 to 80 mg/ml, in particular 30 to 60 mg/ml, in particular 50 mg/ml, in relation to the volume of water or buffer in the reaction medium (wet weight to total volume of water or buffer).

In a particularly preferred embodiment, the reaction in step y) takes place without cryogenic conditions.

In a preferred embodiment, the reaction time of the reaction in step y) is 1 to 150 h, in particular 10 to 100 h.

In a preferred embodiment of the present invention, step y) is carried out under mechanical agitation, in particular shaking or stirring.

In a preferred embodiment of the present invention, step y) is carried out under mechanical agitation, in particular shaking or stirring, preferably with 1 to 5000 rpm, preferably 10 to 3000 rpm, preferably 100 to 800 rpm, preferably 200 to 800 rpm.

In a preferred embodiment the reaction in step y) is stopped by adding an organic solvent, in particular ethyl acetate and/or cyclohexane, in particular ethyl acetate and cyclohexane in a 1 to 1 (v/v) mixture.

The present invention also relates to 1-(R)-cis-PMD comprising composition, preferably obtainable by the process according to the present invention.

In a preferred embodiment, the composition obtainable according to the present invention comprises 1-(R)-cis-PMD with a diastereomeric excess of at least 70, at least 80 or preferably at least 90 % (each in de (%) and based on 1-(R) cis and trans-PMD).

In a preferred embodiment, the composition obtainable according to the present invention comprises 1-(R)-cis-PMD with an enantiomeric excess of at least 90, or preferably at least 95 %, preferably at least 99% (each in ee (%) and based on 1-(R) cis and 1-(S)-PMD).

In a preferred embodiment, the composition obtainable according to the present invention comprises 1-(R)-cis-PMD with a product selectivity of at least 60, at least 70 or preferably at least 80 % (each in ps (%) and based on all products).

In a preferred embodiment, the composition obtainable according to the present invention comprises 1-(R)-cis-PMD with a diastereomeric excess of at least 70, at least 80 or preferably at least 90 % (each in de (%) and based on 1-(R) cis and trans-PMD) and a product selectivity of at least 60, at least 70 or preferably at least 80 % (each in ps (%) and based on all products).

In a preferred embodiment, the composition obtainable according to the present invention comprises 1-(R)-cis-PMD and further components, in particular by-products, which by-product is in particular the 1-(R)-trans-PMD enantiomer, one of a combination of the 4 stereoisomeric isopulegols according to scheme 3 of example 1 or any combination thereof. Such a composition preferably can be an enantiomeric enriched and/or diastereomeric enriched composition of 1-(R)-cis-PMD. In some embodiments the by-products also comprise (R)-citronellal.

Preferably, the 1-(R)-cis-PMD comprising composition of the present invention further comprises at least one encapsulating agent, in particular cyclodextrin, in particular γ-cyclodextrin. Preferably the encapsulating agent, in particular cyclodextrin, in particular γ-cyclodextrin, is contained in the composition in a concentration of at least 2, at least 5, at least 7 or at least 9 or preferably of at least 10 mM.

According to a preferred embodiment of the present invention, the encapsulating agent, in particular cyclodextrin, in particular γ-cyclodextrin, is contained in the present 1-(R)-cis-PMD comprising composition in a concentration of 2 to 40, of 5 to 30, in particular of 7 to 25, preferably of 10 to 20 mM.

### WT-SHCs, conserved amino acid sequences and functional equivalents

As referred to above, the at least one amino acid exchange provided according to the present invention is an amino acid exchange that replaces the amino acid F at position 365 of the wild type AacSHC of SEQ ID No. 1 or the F at a position in the amino acid sequence of another wild type SHC (WT-SHC) corresponding to F365 in the wild type AacSHC of SEQ ID No. 1 by an amino acid selected from the group consisting of A, C, H, I, M, P, S, T and Y.

In the context of the present invention, the identification of the position of a particular amino acid, preferably F, in an amino acid sequence of a wild type SHC (WT-SHC) corresponding to an amino acid, preferably F, in the wild type AacSHC of SEQ ID No. 1 is preferably done by aligning the WT-SHC amino acid sequence to the amino acid sequence of the wild type AacSHC of SEQ ID No. 1, in particular aligning their sets of conserved amino acids.

Accordingly, the F in a WT-SHC, which is not the wild type AacSHC, corresponding to F365 in the wild type AacSHC of SEQ ID No. 1 is identified by aligning the set of conserved amino acids of the amino acid sequence of the WT-SHC to the set of conserved amino acids of the amino acid sequence of the wild type AacSHC of SEQ ID No. 1.

The amino acid sequence of the wild type AacSHC of SEQ ID No. 1 comprises a set of conserved amino acids, which set of conserved amino acids can be found in identical or corresponding positions in at least one other amino acid sequence, preferably all of them, of a WT-SHC selected from the group consisting of SEQ ID No. 2 to 5, preferably all of them.

In a preferred embodiment of the present invention, the mutant SHC comprises amino acids including a set of conserved amino acids, in particular of the wild type SHC, in particular of the wild type AacSHC, except for the presence of the at least one, in particular one, amino acid mutation of the present invention in the amino acid sequence, in particular in the set of conserved amino acids, in particular of the wild type SHC, in particular of the wild type AacSHC.

Preferably, a set of conserved amino acids in the amino acid sequence of a wild type SHC, in particular wild type AacSHC of SEQ ID No. 1, comprises at least W169, D374, D376, D377, Y420, W489, Y609 and Y612 by reference to the wild type AacSHC of SEQ ID No. 1.

In a preferred embodiment, a set of conserved amino acids in the amino acid sequence of a wild type SHC, in particular wild type AacSHC of SEQ ID No. 1, comprises L22, Q26, G30, W32, A44, E45, L48, Q72, G76, W78, G83, A94, Y95, L98, G102, A113, I117, G121, G122, F129, T130, L134, A135, L136, G138, W142, P146, P149, I163, W169, A170, R171, P185, F217, D222, R237, G259, I261, P263, P281, S309, P310, W312, D313, T314, L316, A320, A336, W339, Q344, G349, D350, W351, P360, G361, G362, A364, F365, N369, Y372, P373, D374, D376, D377, T378, A379, A384, W406, M410, Q411, G415, W417, A419, N424, P433, D436, D442, P443, D447, V448, Q479, A450, Q479, G483, W485, F486, G487, R488, W489, G490, V491, N492, Y493, Y495, G496, T497, A503, L504, A519, W522, Q527, D530, G531, G532, W533, G534, E535, S539, Y540, T552, S554, Q555, T556, W558, A559, L563, A565, G577, L581, Q585, G589, W591, E593, G589, G600, F601, P602, F605, Y609, Y612, F616, P617, A620, L621 and R623 by reference to the wild type SHC of Alicyclobacillus acidocaldarius of SEQ ID No 1.

In a preferred embodiment of the present invention, the mutant SHC comprises amino acids including a set of conserved amino acids, in particular of the wild type SHC, in particular of the wild type AacSHC, except for the presence of the at least one, in particular one, two or three amino acid mutation or mutations of the present invention, wherein the at least one amino acid mutation in the amino acid sequence, in particular in the set of conserved amino acids, of the mutant SHC is a) at least a replacement of the F at position 365 (F365) relative to the wild type AacSHC of SEQ ID No. 1 by an amino acid selected from the group consisting of A, C, H, I, M, P, S, T and Y or b) at least a replacement of the F at a position in an amino acid of the WT-SHC of any one of SEQ ID No. 2 to 5 corresponding to F365 of the wild type AacSHC of SEQ ID No. 1 by an amino acid selected from the group consisting of A, C, H, I, M, P, S, T and Y.

In a preferred embodiment, the functional equivalent of the mutated amino acid sequence is a functional equivalent of the mutated amino acid sequence relative to the wild type AacSHC of SEQ ID No. 1 or relative to the WT-SHC and comprises, in particular consists of, said mutated amino acid sequence relative to the wild type AacSHC of SEQ ID No. 1 or relative to the WT-SHC, which is further characterized by the presence of at least a one further specific amino acid replacement in the mutated amino acid sequence of the mutant SHC of the present invention. The mutated amino acid sequence of the mutant SHC containing said at least one further specific amino acid replacement is the amino acid sequence of the functional equivalent and is termed herein to be a "derivatized mutated amino acid sequence".

In a preferred embodiment of the present invention, the functional equivalent of the mutated amino acid sequence comprises amino acids including a set of conserved amino acids, in particular of the wild type SHC, in particular of the wild type AacSHC, except for the presence of the at least one, in particular one, amino acid mutation of the present invention in the amino acid sequence, in particular in the set of conserved amino acids, and except for the presence of at least one further amino acid mutation in the mutated amino acid sequence, in particular in the set of conserved amino acids.

In a preferred embodiment of the present invention, the functional equivalent of the mutated amino acid sequence comprises amino acids including a set of conserved amino acids, in particular of the wild type SHC, in particular of the wild type AacSHC, except for the presence of the at least one, in particular one, amino acid mutation of the present invention, wherein the at least one, in particular one, amino acid mutation in the amino acid sequence, in particular in the set of conserved amino acids, of the mutant SHC is a) at least a replacement of the F at position 365 (F365) relative to the wild type AacSHC of SEQ ID No. 1 by an amino acid selected from the group consisting of A, C, H, I, M, P, S, T and Y or b) at least a replacement of the F at a position corresponding to F365 of the wild type AacSHC of SEQ ID No. 1 in the amino acid of the wild type SHC, in particular a wild type SHC (WT-SHC) of any one of SEQ ID No. 2 to 5, by an amino acid selected from the group consisting of A, C, H, I, M, P, S, T and Y, and except for the presence of at least one further amino acid mutation in the mutated amino acid sequence, in particular in the set of conserved amino acids.

In addition to that and in a preferred embodiment, the functional equivalent of the mutated amino acid sequence comprising, in particular consisting of, the derivatized mutated amino acid sequence of the mutant SHC has a specified degree of amino acid sequence identity to the mutant SHC or comprises amino acids including a set of conserved amino acids, in particular of the wild type SHC, in particular of the wild type AacSHC, except for the presence of the at least one, in particular one, amino acid mutation of the present invention, wherein the at least one, in particular one, amino acid mutation in the amino acid sequence, in particular in the set of conserved amino acids, of the mutant SHC is a) at least a replacement of the F at position 365 (F365) relative to the wild type AacSHC of SEQ ID No. 1 by an amino acid selected from the group consisting of A, C, H, I, M, P, S, T and Y or b) at least a replacement of the F at a position corresponding to F365 of the wild type AacSHC of SEQ ID No. 1 in the amino acid of the wild type SHC, in particular a wild type SHC (WT-SHC) of any one of SEQ ID No. 2 to 5, by an amino acid selected from the group consisting of A, C, H, I, M, P, S, T and Y, and except for the presence of at least one further amino acid mutation in the mutated amino acid sequence, in particular in the set of conserved amino acids.

In a preferred embodiment, the functional equivalent of the mutated amino acid sequence of the mutant SHC has an amino acid sequence identity from 38,0 to 99,9 % to the amino acid sequence of the mutated amino acid sequence of the mutant SHC, in particular 40,0 to 99,9 %, in particular 45,0 to 99,9 %, in particular 50,0 to 99,9 %, in particular 60,0 to 99,9 %, in particular 70,0 to 99,9 %, in particular 80,0 to 99,9 %, in particular 90,0 to 99,9 %, in particular 95,0 to 99,9 %, in particular 98,0 to 99,9 %.

The sequence identity is determined using the algorithm of Pearson and Litman (Proc. Natl. Acad. Sci USA, 85 (8), 1988, 2444 - 2448). The identity is an identity of the amino acids based on the entire length of the amino acid sequences.

In a particularly preferred embodiment, the functional equivalent of the mutated amino acid sequence comprises, in particular consists of, a derivatized mutated amino acid sequence with a length of at least 400, preferably at least 500, preferably at least 550, preferably at least 600 amino acids, preferably at least 700 amino acids, preferably at least 800 amino acids. Preferably, according to the invention, the sequence identity of the derivatized mutated amino acid sequence to the mutated amino acid sequence of the mutant SHC is given over the entire length of the derivatized mutated amino acid sequence.

### Biocatalyst and its preparation

In further preferred embodiments, the present invention also relates to a nucleic acid sequence or nucleic acid molecule, in particular polynucleotide sequence, in particular DNA sequence, encoding a mutated SHC protein according to the invention, in particular encoding a mutant SHC protein with a mutation at F365 and L607.

The nucleic acid sequence, in particular DNA sequence, according to the invention may additionally have, preferably in reading frame to the DNA sequence encoding the protein according to the invention, further DNA sequences which encode, for example, signal peptides, in particular secretion signals. Such additional DNA sequences may also encode amino acids or amino acid sequences that enable or facilitate purification of proteins according to the invention.

In further preferred embodiments, the present invention also relates to an expression cassette, comprising a nucleic acid sequence according to the invention.

In a preferred embodiment, the expression cassette may contain in addition to the nucleic acid sequence of the present invention, regulatory elements such as promoters, terminators, polyadenylation signals, enhancers or other regulatory elements. Preferably, the nucleic acid sequence of the present invention is operably linked to a promoter and optionally an enhancer enabling transcription of the nucleic acid molecule.

In further preferred embodiments, the present invention also relates to a vector, in particular a plasmid or phase, comprising a nucleic acid sequence, or an expression cassette according to the invention.

In a particularly preferred embodiment, the vector may comprise, in addition to a nucleic acid sequence according to the invention, further structural and/or functional units, for example regulatory elements such as promoters, enhancers, terminators or other functional units such as resistance genes or linkers.

In further preferred embodiments, the present invention also relates to a host cell, in particular recombinant host cell, comprising a vector, in particular a plasmid, or an expression cassette or a nucleic acid sequence according to the invention.

In a further preferred embodiment, the present invention also relates to a host cell, which also comprises a vector, in particular a plasmid, or an expression cassette, each of them comprising a nucleic acid sequence, which nucleic acid sequence codes for a mutant SHC being of use for the presently provided process to obtain cis-4.

In a preferred embodiment of the present invention, the host cell comprising a vector, in particular a plasmid, or an expression cassette or a nucleic acid sequence, is obtained by transfecting the host cell with the vector, expression cassette and/or nucleic acid sequence by a conventional means, for instance heat-shock, ballistic, electrical and/or chemical methods.

The host cell of the present invention is able to express the mutant SHC or the functional equivalent of the present invention or used for the present process and, thus, under the appropriate culture conditions, produces the mutant SHC or the functional equivalent.

In the context of the present invention, a host cell, which produces a mutant SHC or a functional equivalent, preferably secretes and/or contains mutant SHC or functional equivalent, is also considered to be a host cell of the present invention.

In a particularly preferred embodiment, the host cell may be a eukaryotic or prokaryotic host cell. In a preferred embodiment, the host cell is a microorganism, fungal cell, plant cell, animal cell or human cell. In a particularly preferred embodiment, the prokaryotic host cell may be a bacterial host cell, preferably E. coli.

In a particularly preferred embodiment, the eukaryotic host cell may be an animal cell, in particular mammalian or insect cell, plant cell or fungal cell.

The present invention also provides a process for producing a mutant SHC or a functional equivalent by recombinant means, in particular said process encompassing the expression of nucleic acid sequences encoding a mutant SHC or a functional equivalent in a host cell. Advantageously, not only the mutant SHC or the functional equivalent obtained and isolated in this process, but particularly the mutant SHC or the functional equivalent expressed in the host cell and remaining physically associated with the host cell, in particular intracellularly located or membrane bound, shows a surprisingly high biocatalytic performance, in particular high turnover frequency and/or short reaction time. Thus, the present invention provides an isolated mutant SHC or a functional equivalent as well as a mutant SHC or a functional equivalent which is physically associated with the host cell, in particular intracellularly located or membrane bound, in which it is expressed.

Thus, in a preferred embodiment, the mutant SHC or the functional equivalent remains physically associated with a host cell, in particular intracellularly located in the host cell which expressed the mutant SHC or the functional equivalent and is obtained together with the host cell, preferably as a viable host cell or a non-viable host cell.

Preferably the present invention relates to a mutant SHC or a functional equivalent which is physically associated with the host cell, in particular membrane bound to a host cell which expressed the mutant SHC or the functional equivalent, which means that the mutant SHC or the functional equivalent is located in the cell membrane of the host cell.

Preferably, the present invention, also provides processes according to which the host cell containing the expressed mutant SHC or functional equivalent and/or with which the expressed mutant SHC or functional equivalent is physically associated, in particular intracellularly located or membrane bound, is further processed so as to produce host cell lysates, host cell extracts, freeze-dried host cell or cell fractions, in particular cell membrane fragments, cytoplasmic fractions or both, preferably all of them containing the expressed mutant SHC or functional equivalent and/or with which the expressed mutant SHC or functional equivalent is physically associated, in particular intracellularly located or membrane bound, and, therefore, containing the enzymatic activity of a mutant SHC or a functional equivalent.

Preferably, the viable and non-viable host cells, host cell extracts, host cell lysates, freeze-dried host cells or host cell fractions, in particular host cell membrane fragments, show the present advantageous high enzymatic activity of a mutant SHC or a functional equivalent and are considered herein together with the mutant SHC or the functional equivalent itself as "biocatalyst" of the present invention.

In a preferred embodiment of the present invention, the biocatalyst of the present invention is the cell membrane fragments of the host cell.

In a preferred embodiment, the cell membrane fragments of the host cell according to the present invention exhibit the enzymatic activity of a mutant SHC or a functional equivalent.

In a furthermore preferred embodiment, the host cell containing the mutant SHC or the functional equivalent, in particular in its cell membrane, is a viable cell, in particular whole cell.

In a further preferred embodiment, the host cell containing the mutant SHC or the functional equivalent is an inactivated or a dead cell, in particular whole cell.

In a furthermore preferred embodiment of the present invention, the biocatalyst containing the mutant SHC or the functional equivalent is a host cell extract, freeze-dried host cell, a host cell lysate or a host cell fraction, in particular cell membrane fragments.

The biocatalyst of the present invention may be in form of a powder or in liquid or semi-liquid form, for instance in form of a suspension.

In further preferred embodiments, the present invention thus relates to a process for producing a biocatalyst, in particular host cell or mutant SHC or the functional equivalent, that means protein according to the invention, comprising the following steps:
a) providing a host cell comprising a nucleic acid sequence encoding a mutant SHC or a functional equivalent,
b) cultivating the host cell of step a) in a culture medium under conditions, which allow the expression of the mutant SHC or the functional equivalent and
c) obtaining the biocatalyst, in particular the expressed mutant SHC or functional equivalent or the host cell containing the expressed mutant SHC or functional equivalent.

In a particularly preferred embodiment of the present invention, the expressed mutant SHC or functional equivalent obtained in step c) is obtained by extraction using a detergent, in particular CHAPS, CHAPS hydrate, Triton X-100, or Tween, in particular Tween 80.

In a particularly preferred embodiment of the present invention, the host cell, the culture medium, and the conditions allowing the expression of the mutant SHC or the functional equivalent provided in steps a) and b) are such, that the mutant SHC or the functional equivalent is secreted by the host cell and obtained in the culture medium, from which it can be isolated by methods known in the art. Thus, the invention preferably relates to a process comprising process steps a), b) and c), wherein in a step d) following step c) the mutant SHC or the functional equivalent is isolated from the culture medium, preferably in pure and/or homogenous form.

In a preferred embodiment, the mutant SHC or the functional equivalent is, thus, obtained in isolated form, preferably in pure and/or homogenous form.

Accordingly, in a preferred embodiment of the present invention, the expressed mutant SHC or functional equivalent obtained in step c) is subjected to a subsequent step d) for isolating a mutant SHC or a functional equivalent from the culture medium.

In a further preferred embodiment, the host cell, the culture medium and the conditions allowing the expression of the mutant SHC or the functional equivalent provided in steps b) and c) are such, that the mutant SHC or the functional equivalent remains physically associated with the cell, in particular intracellularly located or membrane bound.

Thus, in a furthermore preferred embodiment, the expressed mutant SHC or functional equivalent obtained in step c) is contained in and/or physically associated with the host cell, in particular in its cell membrane.

Thus, in a further preferred embodiment, the host cell, the culture medium and the conditions allowing the expression of the mutant SHC or the functional equivalent provided in steps b) and c) are such, that the mutant SHC or the functional equivalent remains physically associated with the host cell, in particular intracellularly located or membrane bound, and is obtained in step c) together with the host cell, preferably as a viable host cell or a non-viable host cell.

In a preferred embodiment, obtaining the expressed mutant SHC or functional equivalent contained in or physically associated with the host cell, in particular intracellularly located or membrane bound, according to step c) is followed by a subsequent step e) for isolating the host cell from the culture medium.

In a preferred embodiment, the host cell isolated in step e) can be disrupted by a subsequent process step f), in particular using pressure or shear forces, lysis by detergents or acids, thermal treatment, osmotic shock, ultrasound, electrical digestion methods or other known methods.

Thus, in a furthermore preferred embodiment of the present invention, the host cell containing the expressed mutant SHC or functional equivalent and/or with which the mutant SHC or the functional equivalent is physically associated, in particular intracellularly located or membrane bound, obtained in step c) or e) is further processed in step f) so as to produce host cell lysates, host cell extracts, freeze-dried host cell or cell fractions, in particular cell membrane fragments, cytoplasmic fractions or both, preferably all of them containing the expressed mutant SHC or functional equivalent and/or the mutant SHC or the functional equivalent is physically associated with all of them, in particular intracellularly located or membrane bound, and, therefore, containing the enzymatic activity of a mutant SHC or a functional equivalent.

In a preferred embodiment of the present invention, step f) results in the formation of a host cell extract, a host cell lysate, freeze-dried host cells or host cell fractions, which host cell fractions are preferably host cell membrane fragments.

In a preferred embodiment of the present invention, the biocatalyst of the present invention may be combined with a conventional biocatalyst carrier such as alginate.

In further preferred embodiments, the present invention also relates to the use of a biocatalyst, in particular mutant SHC or the functional equivalent, a nucleic acid molecule, an expression cassette, a vector or a host cell according to the present invention for at least the cyclohydration of (R)-citronellal to 1-(R)-cis-PMD.

In preferred embodiments, the present invention relates to a bioreactor, comprising at least one biocatalyst of the present invention, in particular a biocatalyst of the present invention associated with a carrier, preferably the biocatalyst and/or the biocatalyst supported on the carrier are contained in a reaction medium in the bioreactor.

### Definitions

In the context of the present invention, the terms 1-(R)-cis-PMD, (1R)-cis-PMD, cis-4 and (1R)-(+)-cis-PMD and 1R-(+)-cis-p-Menthane-3,8-diol are also meant to designate the same stereoisomer of PMD (para-menthane-3,8-diol, CAS Registry Number® 42822-86-6; 1R-cis-PMD, CAS Registry Number® 92471-23-3).

In the context of the present invention, the performance index is calculated by:
(de multiplied by 1,5) plus ps plus rc= performance index
The product selectivity (ps) is determined as follows: area_{1R-cis-PMD}/(area_{products})
The diastereomeric excess (de) is determined as follows: area_{1R-cis-PMD}-area_{1R-trans-PMD}/ (c_{1R-cis-}PMD+C_{1R-trans-PMD})
The relative average conversion (rc) is determined as follows: area_{products}/(area_{substrate+products})
The enantiomeric excess (ee) is determined as follows: area_{1R-cis-PMD}-area_{1S-cis-PMD}/(area_{1R-cis-PMD}+area_{1S-cis-PMD})
The measurements of the ps, de, rc and ee represented by the indicated areas were performed by gas chromatography (GC), in particular GC-FID (flame ionization detector), in particular such as specified in example 3, section "Analytics".

In the context of the present invention, the phenylalanin (F) in the amino acid position of the WT -SHC corresponding to phenylalanin (F) at position 365 in the wild type AacSHC of SEQ ID No. 1 is identified by aligning the set of conserved amino acids of the wild type-AacSHC of SEQ ID No. 1 to the set of conserved amino acids in the wild type-SHC and thereby identifying the amino acid position of the phenylalanin (F) of the wild type-SHC corresponding to the F at position 363 in the wild type AacSHC of SEQ ID No. 1.

In the context of the present invention, the position of an amino acid in an amino acid sequence of a WT-SHC corresponding to an amino acid of wild type AacSHC is identified by aligning the amino acid sequence of the WT-SHC, preferably selected from the group consisting of SEQ ID Nos 2 to 5, to the amino acid sequence of the wild type AacSHC (SEQ ID No. 1), in particular to the set of conserved amino acids of the wild type AacSHC (SEQ ID No. 1), in particular using Clustal Omega.

In the context of the present invention, a conserved amino acid means an amino acid of an amino acid sequence of one species which is identical to an amino acid in one or more amino acid sequences of at least one other species in the corresponding position of the at least one other amino acid sequence, which corresponding position preferably is determined by sequence alignment, herein also termed aligning.

In the context of the present invention, a set of conserved amino acids is composed of at least 2 conserved amino acids which may be consecutive or non-consecutive, that means interrupted by an amino acid sequence gap, and which at least 2 amino acids are identical to at least 2 amino acids in the corresponding positions of at least one other amino acid sequence being aligned.

In the context of the present invention, the sequence alignment, is a way of arranging the sequences of amino acids to identify regions of identity. Aligned sequences of amino acids are represented as rows within a matrix, in particular and preferably the matrix presented in table 5. Gaps are, if necessary, inserted between the identical amino acids so that identical amino acids are aligned in successive columns. In a preferred embodiment of the present invention, the sequence alignment is done using the software Clustal Omega. This online tool is a bioinformatics sequence analysis application, which is free to use.

In the context of the present invention, the set of conserved amino acids of the wild type AacSHC (SEQ ID No. 1) is identified by aligning the amino acid sequence of the wild type AacSHC (SEQ ID No. 1) with at least one other amino acid sequence of a WT-SHC, preferably at least 2, 3, or 4 other amino acid sequences of a WT-SHC, preferably selected from the group consisting of SEQ ID Nos 2 to 5, in particular using Clustal Omega.

In the context of the present invention, the term "a set of conserved amino acids at positions X by reference to the wild type AacSHC of SEQ ID No. 1" means that the specific amino acids at positions X as referred to can be found at the indicated positions in the amino acid sequence of the AacSHC (SEQ ID No. 1), and at the same or a corresponding position in aligned amino acids sequences of a different WT-SHC.

In the context of the present invention, a "functional equivalent of a mutated amino acid sequence of a protein of the present invention", that means of a mutant SHC, is a variant of the mutant SHC which variant comprises a derivatized mutated amino acid sequence comprising the mutated amino acid sequence of the mutant SHC, and at least one amino acid difference to the mutated amino acid sequence in at least one amino acid position and/or in the order of the amino acids, for instance comprises at least one additional amino acid, lacks at least one amino acid or comprises at least one amino acid inversion.

In the context of the present invention, a functional equivalent of the mutant SHC exhibits the same functions as identified herein for the mutant SHC, in particular in terms of defining qualitatively and quantitatively the enzymatic activity, in particular stereoselectivity and product selectivity. Preferably the functional equivalent of the mutant SHC has at least one further amino acid mutation in the mutated amino acid sequence of the mutant SHC. Preferably, the functional equivalent comprises a derivatized mutated amino acid sequence.

In the context of the present invention, "stereoisomers" are isomers in which the bonds are the same but the relative positions of the atoms in space differ. Stereoisomers is the generic term for enantiomers and diastereomers.

In the context of the present invention, "enantiomer" is understood to mean one of two chiral compounds of the same constitution, wherein the two chiral compounds behave like image and mirror image.

In the context of the present invention, "diastereomer" is understood to mean one of at least two chiral compounds of the same constitution, wherein the at least two chiral compounds do not behave like image and mirror image. Hence, they occur when two or more stereoisomers of a compound have different configurations at one or more, but not all, of the equivalent stereocentres. When two diastereomers have more than one stereocentre but differ from each other at only one stereocentre they are epimers (epi).

In the context of the present invention, diastereomers differ from enantiomers in that the latter are pairs of stereoisomers that differ in all stereocentres and are therefore mirror images of one another. Enantiomers of a compound with more than one stereocentre are also diastereomers of the other stereoisomers of that compound that are not their mirror image.

In the context of the present invention, "compound" is understood to mean a molecule or several identical molecules.

In the context of the present invention, the term "nucleic acid molecule" is understood to refer to an isolated polynucleotide or, if the nucleic acid molecule is integrated into a stretch of another polynucleotide, for instance into an expression cassette or into a vector, the term "nucleic acid molecule" refers to such an integrated stretch of polynucleotides. Thus, the term "nucleic acid molecule" can be interchangeably used to the term "nucleic acid sequence".

Nucleic acid molecules according to the present invention may be DNA or RNA, preferably DNA.

In the context of the present invention the amino acids are presented in the one-letter-code, wherein A is alanine, R is arginine, N is asparagine, D is aspartic acid, C is cysteine, E is glutamic acid, Q is glutamine, G is glycine, H is histidine, I is isoleucine, L is leucine, K is lysine, M is methionine, F is phenylalanine, P is proline, S is serine, T is threonine, W is tryptophan, Y is tyrosine and V is valine.

In the context of the present invention, specific mutants of the SHC according to the invention are identified by indicating the type of amino acid replaced in an amino acid sequence, for example SEQ ID No. 1, in the one-letter code, followed by the positional indication, with respect to SEQ ID No. 1, at which the replaced amino acid is located, and then followed by the indication of the type of the different amino acid replacing the replaced amino acid in the one-letter code. For example, the designation F365A means that in SEQ ID No. 1 with the wild-type AacSHC sequence, the amino acid F present at position 365 is replaced by another amino acid, namely A.

In the context of the present invention, the term "obtaining a substance, for instance protein, or composition" is understood to mean that the substance, for instance protein, or composition produced in a preceding step by reacting reactants is made available from the respective culture or reaction medium or solvent, in particular is isolated from it, in particular obtaining a composition is therefore to be understood preferably as concentrating or filtering off, in particular isolating, the desired composition or substance. The processes used for this can be physical, chemical processes or biological processes.

In the context of the present invention, the term "and/or" is understood to mean that all members of a group connected by the term "and/or" are represented both cumulatively with respect to each other in any combination, and alternatively with respect to each other. Exemplarily, for the expression "A, B and/or C", the following disclosure is to be understood thereunder: i) (A or B or C), or ii) (A and B), or iii) (A and C), or iv) (B and C), or v) (A and B and C), or vi) (A and B or C), or vii) (A or B and C), or viii) (A and C or B).

In the context of the present invention the use of the brackets "{ }" is not understood to mean that the process steps as referred to within the brackets do not belong to the invention but merely serves to indicate a combination of process steps of the invention.

In the context of the present invention, individual components or constituents of a composition or of one of its components, determined quantitatively in relative form, in particular in percentages, preferably add up to 100% by weight or molar amount of the respective composition referred to or of the composition or, if referred to, of a component thereof, unless otherwise stated.

If in connection with the present invention a "presence", a "containing", a "comprising" or a "content" of a component is expressly mentioned or implied, this means that the respective component is present, in particular is present in a measurable amount.

If in the context of the present invention a "presence", a "containing", a "comprising" or a "content" of a component in an amount of 0 [unit], in particular mg/kg, µg/kg or % by weight, is expressly mentioned or implied, this means that the respective components are not present in a measurable amount, in particular are not present.

Where quantitative indications, in particular percentages, of components of a product or composition are given in the context of the present invention, these add up to 100% of the composition and/or product together with the other explicitly stated or further components obvious to a person skilled in the art of the composition or product, unless explicitly stated otherwise or obvious to a person skilled in the art.

In the context of the present invention, the term "at least one" is understood to mean a quantity expressing a number of 1 or 2 or 3 or 4 or 5 or 6 or 7 or 8 or 9 or 10 and so on. In a particularly preferred embodiment, the term "at least one" may represent exactly the number 1. In another preferred embodiment, the term "at least one" may also mean 2 or 3 or 4 or 5 or 6 or 7.

The number of decimal digits indicated corresponds to the precision of the measurement method used in each case.

If, in the context of the present invention, the first and second decimal digits or the second decimal digit are/is not indicated for a number, this/these shall be set as zero.

In the context of the present invention, the terms "comprising", "containing" and "having" are understood to mean that in addition to the elements explicitly covered by these terms, other elements not explicitly mentioned may be added. In the context of the present invention, it is also understood by these terms that only the explicitly mentioned elements are covered and that no further elements are present. In this particular embodiment, the meaning of the terms "comprising", "containing" and "having" is synonymous with the term "consisting of". Furthermore, the terms "comprising", "containing" and "having" also cover compositions which, in addition to the explicitly mentioned elements, also contain further elements which are not mentioned but which are of a functional and qualitatively subordinate nature. In this embodiment, the terms "comprising", "containing" and "having" are synonymous with the term "consisting essentially of". The term "consisting of' is understood to refer only to the explicitly mentioned elements and excludes the presence of any other element than that being explicitly mentioned.

Further preferred embodiments of the present invention are apparent from the dependent claims.

The present invention will be explained in more detail in the following examples and the accompanying figures both of which are not to be understood as limiting.

The sequence listing shows, wherein SEQ ID Nos 6 to 10 represent preferred embodiments of the present invention:
SEQ ID No 1: WT-SHC of Alicyclobacillus acidocaldarius (Aac)
SEQ ID No 2: WT-SHC of Streptomyces coelicolor (ScoSHC)
SEQ ID No 3: WT-SHC of Zymomonas mobilis (ZmoSHC1)
SEQ ID No 4: WT-SHC of Zymomonas mobilis (ZmoSHC2)
SEQ ID No 5: WT-SHC of Thermosynechococcus elongatus (TelSHC)
SEQ ID No 6: Mutation F365A/Y420W/G600F WT-SHC of Aac
SEQ ID No 7: Mutation F365A/L607G in WT-SHC of Aac
SEQ ID No 8: Mutation F365A/L607G/G600F in WT-SHC of Aac
SEQ ID No 9: Mutation in F365A/L607M/G600F WT-SHC of Aac
SEQ ID No 10: Mutation F365A in WT-SHC of Streptomyces coelicolor.
SEQ ID No 11: DNA sequence (Aac) of SEQ ID No. 1.

The figures show:
Figure 1: The three controls buffer, AXAA-Knock-out and Empty vector demonstrate that the reaction is also catalyzed by the E. coli background.
Figure 2: Biotransformations using lyophilized E. coli cells expressing the corresponding SHC. (A) Comparison of five SHC WTs and the triple variant F365A/Y420W/G600F from the Bastian study. (B) Deconvolution of the triple variant from the Bastian study.

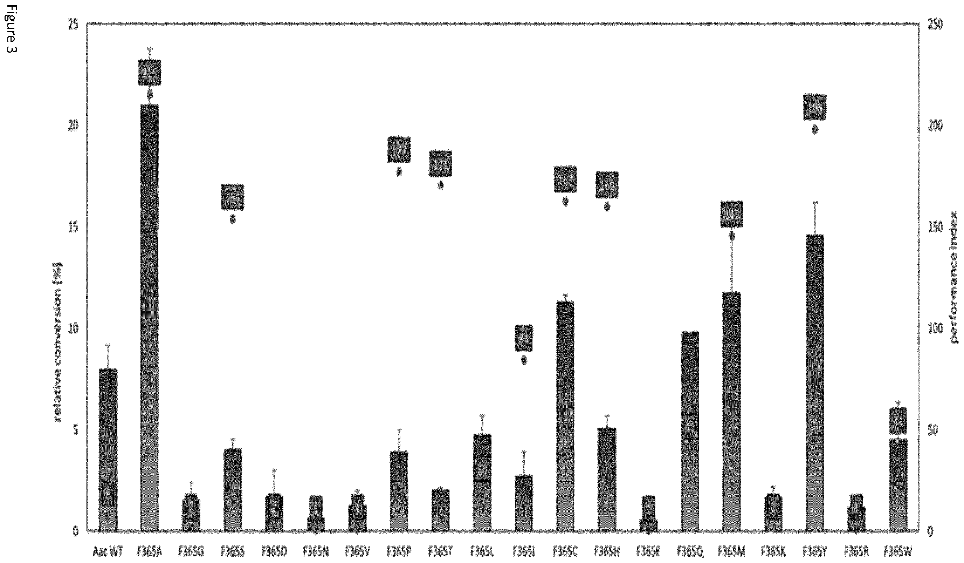

Figure 3: Saturation of F365 and biotransformations using lyophilized E. coli cells thereof.
Figure 4: (A) Evolution of AacSHC towards high-performance cis-4 production. (B) F365A Transfer variant, second generation SHC variants. Amino acid counting corresponds to Aac.
Figure 5: Determination of de and ee of (A) the commercial AntiBrumm naturel^{®}, (B) the empty vector and (C) the SHC variant F365A/L607G/G600F on the Shimadzu GC-FID. (D) Calculation of de and ee.

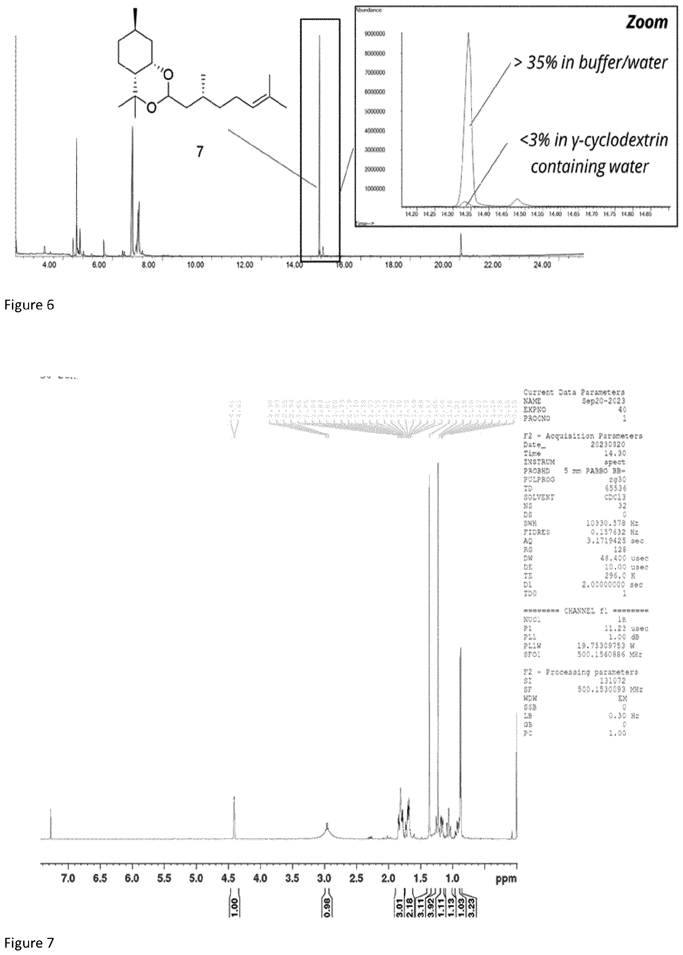

Figure 6: GC chromatogram of the upscaled biotransformation using either water as a buffer or water including substrate-equimolar amount of encapsulation agent γ-cyclodextrin. Encapsulation reduces the acetalization by >90%.
Figure 7: 1H-NMR spectrum

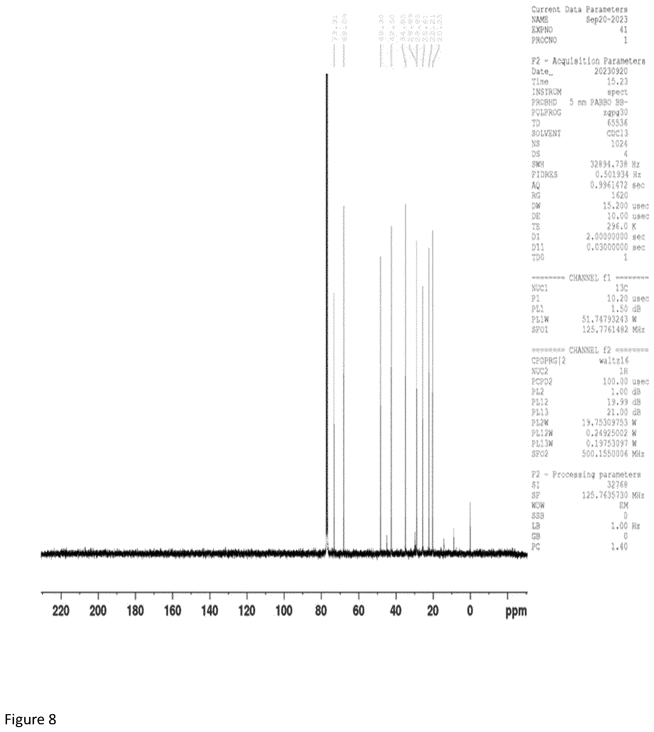

Figure 8: 13C-NMR spectrum

### Example 1

### SHC library evaluation, engineering and evolution of SHC towards cis-4 production

Several SHC wildtypes (WTs) and variants thereof were evaluated in the biotransformation of (R)-citronellal 5 using lyophilized E. coli whole cells expressing the SHC. The candidates were: SHC WTs originating from Alicyclobacillus acidocaldarius (Aac), Thermosynechococcus elongatus (Tel), Zymomonas mobilis 1 (Zmo1), Zymomonas mobilis 2 (Zmo2), and Streptomyces coelicolor (Sco) and the AacSHC triple variant F365A/Y420W/G600F from the aforementioned study (Bastian et al., 2017). Potential cyclization products are shown in Scheme 3, where molecule 6 represents all four potential isopulegols. The variants were ranked by their performance index, which was calculated by the sum of diastereomeric excess (de) multiplied by 1.5, the product selectivity (ps) that describes the GC area of 1R-cis-4 share of the GC area of all occurring cyclization products, and the average GC conversion (rc). This performance index is intended to ensure that the enzymes with the highest diastereomer ratio prevail. A summary of all data is given in Table 1. 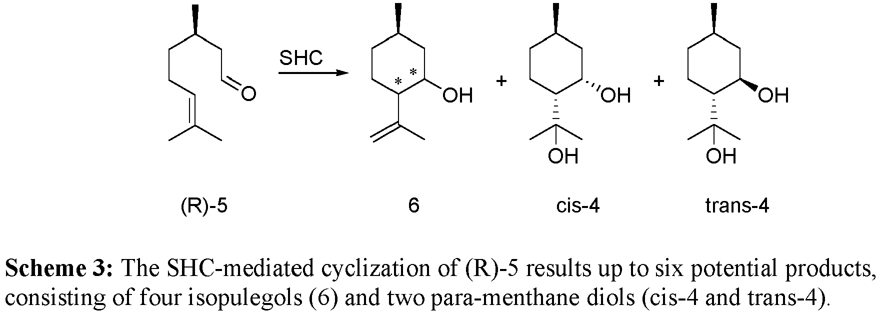 The empty vector control lacking the SHC gene, as well as the knock-out variant (A₃₇₄XA₃₇₆A₃₇₇), which has all three catalytically active aspartates substituted by alanines also generated up to 12% of cis-4. Therefore, the AXAA-knock-out was used as a threshold.

The experimental details, in particular materials and methods, are given in Example 3.

**Table 1: A summary of all variants generated during this study and data retrieved from the current state-of-the-art as well as the commercial product.**

| **Enzyme** | **Average relative conversion [%]** | **de [%]** | **product selectivity [%]** | **performance index** |
|---|---|---|---|---|
| Aac WT | 8 | 0 | 0 | 8 |
| Sco WT | 15 | 0 | 0 | 15 |
| Tel WT | 2 | 0 | 0 | 2 |
| Zmol WT | 5 | 0 | 0 | 5 |
| Zmo2 WT | 0 | 0 | 0 | 0 |
| F365A Y420W | 11 | 72 | 81 | 200 |
| G600F | | | | |
| F365A G600F | 12 | 74 | 75 | 198 |
| F365A Y420W | 29 | 91 | 70 | 235 |
| F365A | 21 | 88 | 62 | 215 |
| F365G | 2 | 0 | 0 | 2 |
| F365S | 4 | 92 | 11 | 154 |
| F365D | 2 | 0 | 0 | 2 |
| F365N | 1 | 0 | 0 | 1 |
| F365V | 1 | 0 | 0 | 1 |
| F365P | 4 | 84 | 47 | 177 |
| F365T | 2 | 82 | 46 | 171 |
| F365L | 5 | 0 | 15 | 20 |
| F365I | 3 | 41 | 20 | 84 |
| F365C | 11 | 51 | 75 | 163 |
| F365H | 5 | 62 | 62 | 160 |
| F365E | 1 | 0 | 0 | 1 |
| F365Q | 10 | -14 | 10 | 41 |
| F365M | 12 | 62 | 40 | 146 |
| F365K | 2 | 0 | 0 | 2 |
| F365Y | 15 | 82 | 61 | 198 |
| F365R | 1 | 0 | 0 | 1 |
| F365W | 5 | -23 | 4 | 44 |
| Sco F365A | 11 | 61 | 35 | 137 |
| F365A L607G | 60 | 91 | 73 | 270 |
| F365A L607M | 24 | 89 | 66 | 224 |
| F365A G600F | 72 | 94 | 72 | 285 |
| L607M | | | | |
| F365A G600F | 95 | 94 | 81 | 317 |
| L607G | | | | |
| AntiBrumm naturel^{®} state-of-the-art (Borrego *et al.,* 2021) | - | 18 | 35 | - |
| | 80 | 76 | 68 | 262 |

| | | | | |
|---|---|---|---|---|
| product selectivity = (area_{1R-cis-4}/area_{allproducts}); performance index = de multiplied by 1,5 + product selectivity + average relative conversion | | | | |

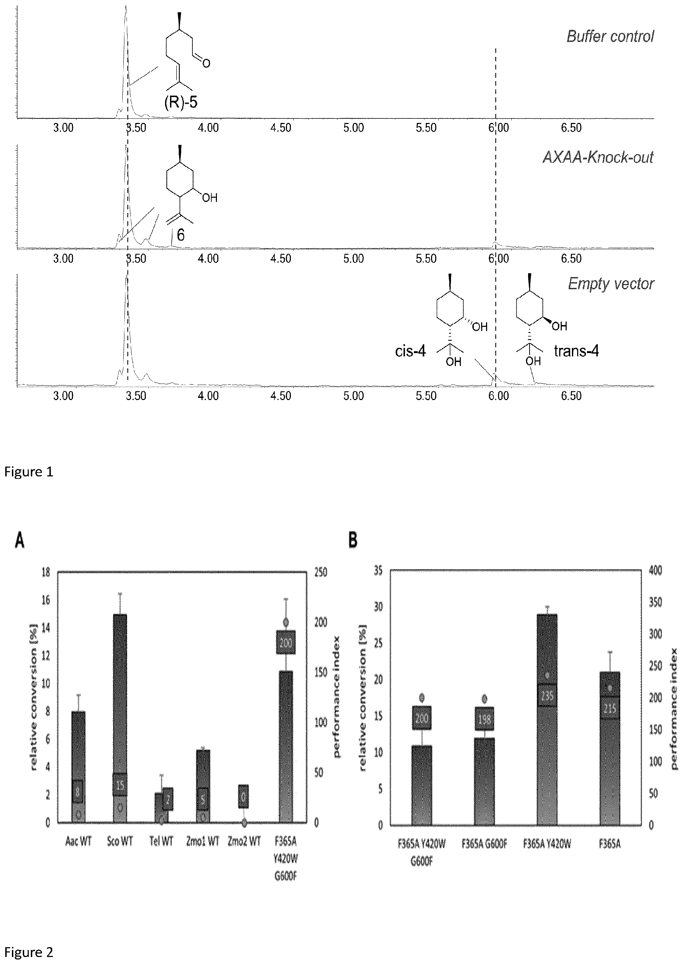

While the SHC WTs did not generate any PMD, the data disclosed that surprisingly the triple variant F365A/Y420W/G600F (SEQ ID No. 6) already generated 81% of the desired cis-4 with 72% de, resulting in a performance index of 200 (Figure 2A). A deconvolution of the triple variant to clarify which mutation has the biggest impact for this result was performed. The data in Figure 2B shows that variants including the F365A mutation perform best with F365A/Y420W and F365A exhibiting 91% and 88% de, respectively.

Position F365 was saturated to elucidate the best substitution. These results showed that the F365A substitution is already the best option.

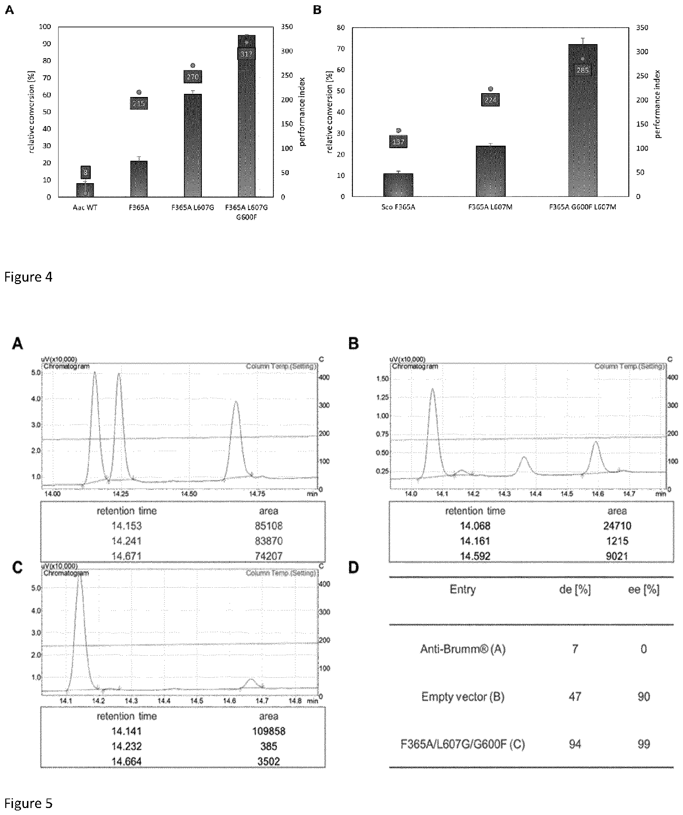

Further saturations at positions L607 and G600 were done. The resulting best hits F365A/L607M and F365A/L607G (SEQ ID No. 7) showed a high-performance index of 224 and 270, as well as a de of 89% and 91%. One more round of site-saturation at position 600 using the new parent F365A/L607G resulted in the variant F365A/L607G/G600F (SEQ ID No. 8) with 94% de, 81% cis-4 selectivity and 97% conversion, resulting in a performance index of 317 (Figure 4A). The ee was determined with >99% (Figure 5). Thus, the evolved enzyme is able to generate a PMD product which has a significantly higher content of cis-4 compared to e.g., commercial AntiBrumm naturel^{®} (cf. Figure 5A and C). Moreover, it supersedes the state-the-art cryogenic cyclization, while acting at ambient temperatures (cf. Table 1).

A recombination of second-best hit F365A/L607M from the first round with G600F led to triple variant F365A/L607M/G600F (SEQ ID No. 9) with the same 94% de but slightly lower cis-4 selectivity of 71. It should be noted that most of the byproduct was the deprotonation product (+)-neo-isopulegol, which could potentially be transformed into cis-4 by subsequent hydration.

Mutation F365A was transferred to homolog Sco, (Figure 4B). Sco F365A (SEQ ID No. 10) generated the desired cis-4 with a performance index of 137. This data shows that other SHC homologs can also be leveraged for cis-4 production.

### Example 2

### Stopping acetal formation by including cyclodextrins

To isolate and characterize the product cis-4 generated with variant F365A/L607G/G600F via NMR, biotransformation in 100 mL scale was conducted. 1 g of E. coli cells expressing the SHC were resuspended in water or water harboring a substrate-equimolar amount of γ-cyclodextrin.

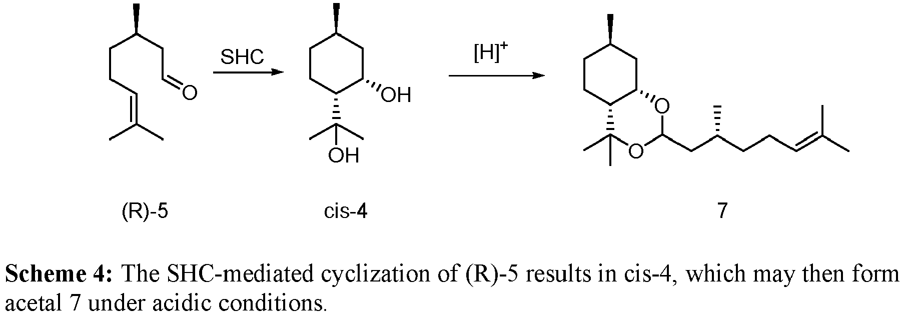

The results demonstrate that the biotransformation including γ-cyclodextrin omitted acetal formation (Figure 5 and scheme 4). Acetal formation is undesired as the acetal has no repellency effect on mosquitos (Borrego *et al.,* 2021). Therefore, including cyclodextrin to the reaction mixture as an encapsulating agent comprises a favorable additive for biotransformation.

### Example 3: Preparation and analysis of mutant SHCs

The preparation and analysis of the mutant SHCs as used and as provided according to the invention is done using the following materials and methods.

**Table 2: List of buffers:**

| **Buffer** | **Ingredients** |
|---|---|
| ddH₂O | - |
| γ-cyclodextrin | 1-10 mM γ-cyclodextrin |

**Table 3: List of media:**

| **Medium** | **Ingredients** |
|---|---|
| Lysogeny broth | 10 g/L tryptone, 10 g/L NaCl, 5 g/L yeast extract |
| Auto-induction medium | 12 g/L tryptone, 24 g/L yeast extract, 11,1 g/L glycerol, 7,6 g/L lactose, 2,9 g/L glucose |

**Table 4: Composition of the PCR mixture:**

| **substance** | **volume [µl]** | **final concentration** |
|---|---|---|
| ddH₂O | 29 | |
| DMSO | 2,5 | |
| KOD Hot Start Buffer (10x) | 5 | 1x |
| dNTPs (2 mM each) | 5 | 250 µm (each) |
| MgSO₄ (25 mM) | 4,5 | 2 mM |
| Template DNA | 1 | 0,5-5 ng/µl |
| Primer forward (10 µM) | 1 | 0,2 µm |
| Primer reverse (10 µM) | 1 | 0,2 µm |
| KOD Hot Start DNA Polymerase | | 1 |

**Table 5: PCR temperature profile:**

| **step** | **Temperature [°C]** | **time [s]** | **cycles** |
|---|---|---|---|
| Initial denaturation | 95 | 120 | 1 |
| Denature | 95 | 30 | |
| Annealing | 60 | 30 | 30 |
| Extension | 70 | 210 | |
| Final extension | 72 | 300 | 1 |

### Material:

- Chemicals: The chemicals used for syntheses, molecular biology and biochemical work have been purchased from Carl-Roth (Karlsruhe, DE), VWR (Pennsylvania, US), Sigma-Aldrich/ Merck (St. Louis, US) and Alfa-Aesar (Ward Hill, US).
- Molecular biological kits: The molecular biological kits for DNA-purification (Zymoclean DNA Clean & Concentrator Kit), Agarose gel-extraction (Zymoclean Gel DNA Recovery Kit) and plasmid isolation (Zyppy^{™}Plasmid Miniprep Kit) were purchased from ZymoResearch (Irvine, US).

### Analytics:

- Nuclear Magnetic Resonance (NMR): ¹H- und ¹³C-NMR spectra were recorded on a Bruker Avance 500 Spectrometer at 500,15 MHz for ¹H- and 125 MHz for ¹³C. The chemical shifts δ are referred to tetramethylsilane (=TMS) in ppm set to 0. All substances were dissolved in CDCl₃ and recorded at room temperature.
- Gas chromatography (GC): GC-MS analyses were performed using an Agilent GC 7820A equipped with a mass spectrometer MSD 5977B and aZB1-HT capillary column (Phenomenex, 30 m x 250 µm x 0,25 µm) and helium as carrier gas with a constant pressure of 14.168 ψ. GC-FID (flame ionization detector) analyses were performed on a Shimadzu GC-2010 Plus equipped with a DB-5 capillary column (Agilent, 30 m x 250 µm x 0,25 µm). Injections (1 µl) were performed in split mode (10:1). GC conversion rates were calculated directly from GC-FID chromatograms by integration-quotient of peak areas derived from substrates and products.
- Chiral Gas chromatography (GC): Chiral GC analyses were performed on a Shimadzu GC-2010 Plus equipped with a Supelco-α-dex capillary column (Supelco, 25 m x 250 µm x 0.25 µm) and hydrogen as carrier gas. Injections (1 µL) were performed in split mode. Stereoselectivities were calculated directly from GC-FID chromatograms by integration-quotient of peak areas derived from products.

### 3.1 Plasmid isolation

Isolation of the plasmid proceeded following to the standard protocol of *Zyppy^{™}Plasmid Miniprep Kit* by ZymoResearch (Zymo Research. Zyppy™ Plasmid Miniprep Kit. Instr. Man. 4037, 1-9, 2014). For the photometric determination of the plasmid DNA concentration, 1 µL was measured on a Nanodrop 1000 (Agilent, Santa Clara, US) at a wavelength of 260 nm.

### 3.2: Site-saturation/-directed mutagenesis of wild type AacSHC (SEQ ID No. 1)

The gene encoding for wild type AacSHC (SEQ ID No. 1 and 11; UniProt: P33247) or a variant based on this gene was cloned into a pET-22b (+) vector system. SacI and NdeI were used as restriction sites. Cloning followed the standard protocol of Novagene's KOD Hot Start DNA Polymerase. The composition of the PCR mixture and the temperature profile is described in table 4 and table 5.

Site-saturation libraries were generated employing the "22c-trick" method (S. Kille, C. G. Acevedo-Rocha, L. P. Parra, Z. G. Zhang, D. J. Opperman, M. T. Reetz, J. P. Acevedo, ACS Synth. Biol. 2013, 2, 83-92.) PCR products were digested with 1 µl DpnI for 4 h at 37 °C, purified by agarose gel electrophoresis and ligated into the pET22b (+) vector by Gibson assembly (D. G. Gibson, L. Young, R. Y. Chuang, J. C. Venter, C. A. Hutchison, H. O. Smith, Nat. Methods 2009, 6, 343-345.). After purification using the DNA Clean & Concentrator^{™}-5 kit the plasmids were transformed via heat-shock method (example 3.3).

Single-point mutated variants were directly transformed after DpnI digest without further purification.

### 3.3: Plasmid transformation via heat-shock method

Chemically competent *E. coli* cells based on rubidium chloride were produced for the transformation of the plasmid DNA. The transformation was carried out under sterile conditions. For site saturation libraries 3 µl (7 µl for single-point mutations) of the purified (crude) PCR product was added to 25 µl XL1-blue competent cells and incubated for 30 min on ice, followed by a heat shock at 42 °C for 105 s with subsequent ice cooling for 3 min. After adding 500 µl of LB medium, the cells were incubated for 40 min at 37 °C and used for inoculation of a 5 ml LB medium (Ampicillin, c_{end}= 100 µg/ml) pre-culture overnight. Single-point variants were plated on petri dishes and grown colonies were picked and grown in 5 ml LB medium. After isolation of the plasmid, sequencing for quality control was performed. Subsequently, transformation into 50 µl BL21 (DE3) was performed using the heat shock method. After regeneration 150 µl were streaked out on an agar plate (Ampicillin, c_{end}= 100 µg/ml) and incubated at 37 °C overnight. For quality control the plasmid was isolated from another 150 µl and sent for sequencing. For site-directed mutant SHCs the PCR product was digested with DpnI overnight and afterwards transformed into XL1-blue competent cells. After regeneration 300 µl were streaked out on an agar plate for single clone picking.

### 3.4: Expression of mutant SHC libraries relative to the wild type AacSHC in 96-DW plates

Individual mutant SHC colonies were picked from generated agar plates and cultivated in 500 µl LB medium (Ampicillin, c_{end}= 100 µg/ml) for 18 to 20 h at 37 °C and 800 rpm. Expression cultures were inoculated with 10 µl of the pre-culture into 1 mL of T-DAB autoinduction medium (Ampicillin, c_{end}= 100 µg/ml) with lactose as the inductor. The cultures were incubated for 20 h at 37 °C and 800 rpm and harvested afterwards (4000 x g, 20 min).

### 3.5: Expression of mutant SHC libraries relative to the wild type AacSHC in 24 DW-plates

Individual mutant SHC colonies were picked from generated agar plates and cultivated in 2 mL LB medium (Ampicillin, c_{end}= 100 µg/ml) for 18-20 h at 37 °C and 180 rpm. Expression cultures were inoculated with 40 µl of the pre-culture into 4 mL of T-DAB autoinduction medium (Ampicillin, c_{end}= 100 µg/ml) with lactose as the inductor. The cultures were incubated for 20 h at 37 °C and 600 rpm and harvested afterwards (4000 x g, 20 min). Quality control was done by SDS-PAGE (see example 3.8)

### 3.6: Lyophilization protocol

Freshly harvested *E. coli* cells including the mutant SHC were transferred to petri dishes and frozen at -80 °C overnight. On the following day the frozen pellets were quickly transferred to a lyophilizer and lyophilized at -80°C at 0,0001 atm for two days. The resulting lyophilized whole cells including mutant SHCs were mortared gently and stored in 50 ml Falcon tubes at room temperature.

### 3.7: Thermolysis purification

(K. Koschorreck, F. Wahrendorff, S. Biemann, A. Jesse, V. B. Urlacher, Process Biochem. 2017, 56, 171-176.)

Harvested or lyophilized *E. coli* cells including the mutant SHC were resuspended in 1 mL Lysis buffer (table 1) and incubated for 60 min at 70 °C. The cell suspension was centrifuged (14000 x g, 1 min) and the supernatant was discarded. As the protein according to the invention is membrane-bound 1 mL 1%-CHAPS buffer (table 1) was added to extract it from the cell pellet by shaking at room temperature for 2 d, 600 rpm. After subsequent centrifugation (14000 x g, 1 min) the supernatant containing the mutant SHC according to the invention was transferred to a new tube followed by SDS-PAGE analysis and determination of protein concentration by using the Nanodrop 1000 (Agilent, Santa Clara, US). Therefore the "Protein A280" mode was chosen with MW= 71439 Da and molar extinction coeffizient ε = 185180 as protein specific data.

### 3.8: SDS-PAGE

After purification and extraction of the mutant SHC, 20 µl of the mutant SHC preparation was mixed with 10 µl SDS loading buffer and heated to 95 °C for 10 min. Afterwards 10 µl of the preparation was loaded on the pre-prepared SDS-PAGE (Expedeon).

### 3.9: Screening of AacSHC libraries via GC-MS

Harvested pellets of *E. coli* cells were frozen overnight and lyophilized afterwards. The dry cells were resuspended in 396 µl H₂O or H₂O containing 5 mM γ-cyclodextrin and transferred to another 96-DW plate equipped with 1,2 mL glass inlets. Afterwards 4 µl (R)-citronellal/DMSO stock solution (R)-citronellal c_{end}= 5 mM) was added directly into the cell suspension including the mutant SHC, the plates were sealed and shaken for 20 h at 30 °C and 600 rpm. In order to stop the reaction 600 µl n-heptane/o-xylol (1:1) was added and the mixture was inverted for 10 min and incubated for 30 min. The plates were centrifuged (4000 x g, 5 min), sealed using PP-sealings and a GC-MS equipped with a PAL-Sampler was used to inject directly from the organic phase. Quantification was made directly from the Total Ion Count chromatogramm by quotient AREA_{product}/(AREA_{substrate}+AREA_{product})*100. In total 90 mutant SHCs per plate were screened. Mutant SHCs were rescreened by expression in 24 DW-plates.

### 3.10: Analysis of mutant SHC's

Mutant SHCs from the 96-DW screening were taken for inoculation of a 5 mL LB pre-culture. Afterwards the plasmids were isolated and transformed for single colony picking. The single colonies were expressed in 24 DW-plates and afterwards harvesting, freezing and lyophilization was conducted. Subsequently, either ddHzO or H₂O containing 5 mM γ-cyclodextrin, was added, the OD set to 20 and 5 mM (R)-citronellal was added. The reactions were carried out at least in technical duplicates. Reactions were stopped by adding n-heptane/o-xylene (1:1) and inversion of the suspension. After two extractions, the resulting organic phase including (1R)-cis-PMD was measured directly over GC-MS. Quantification was made directly from the Total Ion Count chromatogramm by quotient AREA_{product}/(AREA_{substrate}+AREA_{product})*100.

### 3.11: Upscaling of the reaction

For showcasing the scalability of the reaction 1 g of lyophilized *E*. *coli* whole cells harbouring the mutant SHC (F365A/L607G/G600F) (SEQ ID No. 8) were resuspended in either 100 ml ddHzO or 100 ml ddHzO containing γ-cyclodextrin (1,3 g, 1 mmol) and 173 µl (154 mg, 1 mmol), (R)-citronellal was added to the suspension and stirred for 96 h. After full conversion (reaction progress was followed by extracting 10 µl samples and checking via GC) the crude suspension was extracted with ethyl acetate five times. The combined organic phases were concentrated under reduced pressure and dried over NaSO₄ overnight. After filtration and evaporation of the residual solvents, (1R)-cis-PMD was obtained as an oil (120 mg, 0.7 mmol, 70 mol%).

### NMR-Data of (1R)-cis-PMD according to the invention:

**¹H-NMR (CDCl₃, 500 MHz):** δ (ppm) 0,87 (d, J=3Hz, 3H), 0.90-0.97 (m, 1H), 1,05-1,10 (m, 1H), 1,15-1,18 (m, 1H), 1,19-1,24 (m, 1H), 1,22 (s, 3H), 1,36 (s, 3H), 1,65-1,72 (m, 2H), 1,75-1,85 (m, 3H), 2,93-2,98 (m, 1H), 4,4 (d, J =1 Hz, 1H).
**¹³C-NMR (CDCl₃, 125 MHz):** δ (ppm) 20,25 (1C), 22,21 (1C), 25,61 (1C), 28,85 (1C), 34,85 (1C), 42,5 (1C), 48,30 (1C), 68,09 (1C), 73,31 (1C).

### 3.12: Sequence alignment of wild type SHCs

Multiple-sequence alignment was performed with Clustal Omega (F. Sievers, A. Wilm, D. Dineen, T. J. Gibson, K. Karplus, W. Li, R. Lopez, H. McWilliam, M. Remmert, J. Söding, J. D. Thompson, D. G. Higgins, Mol. Syst. Biol. 2011, 7) using the amino acid sequences according to SEQ ID Nos 1 to 5), wherein AacSHC corresponds to SEQ ID No. 1, ScoSHC corresponds to SEQ ID No. 2, ZmoSHC1 corresponds to SEQ ID No. 3, ZmoSHC2 corresponds to SEQ ID No. 4, TelSHC corresponds to SEQ ID No. 5. This program allows the simultaneous alignment of multiple sequences, highlighting conserved amino acids (*) very similar (:), similar sites (.) and differing sites ( ). The result of this alignment is presented in form of a matrix below in table 6:

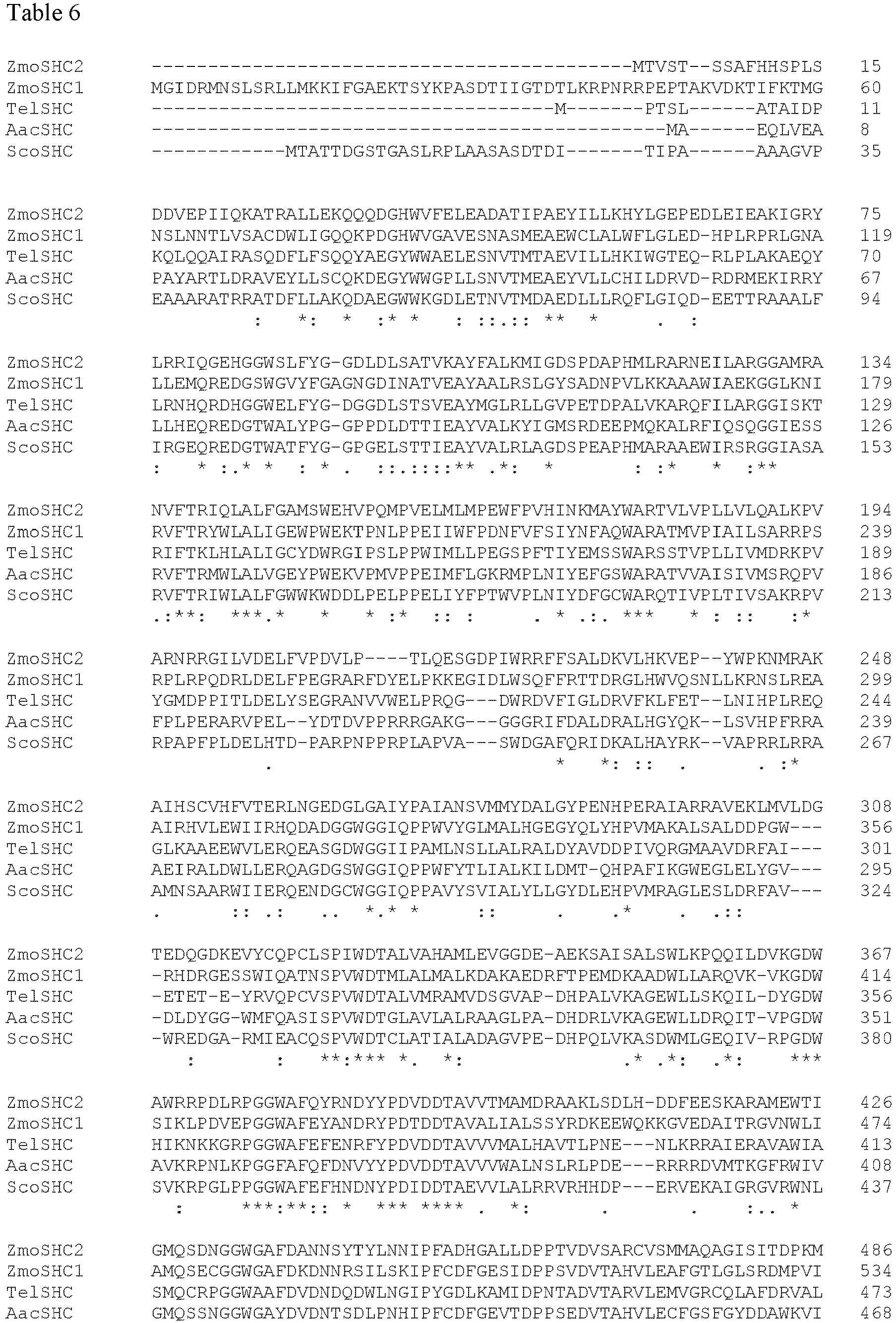

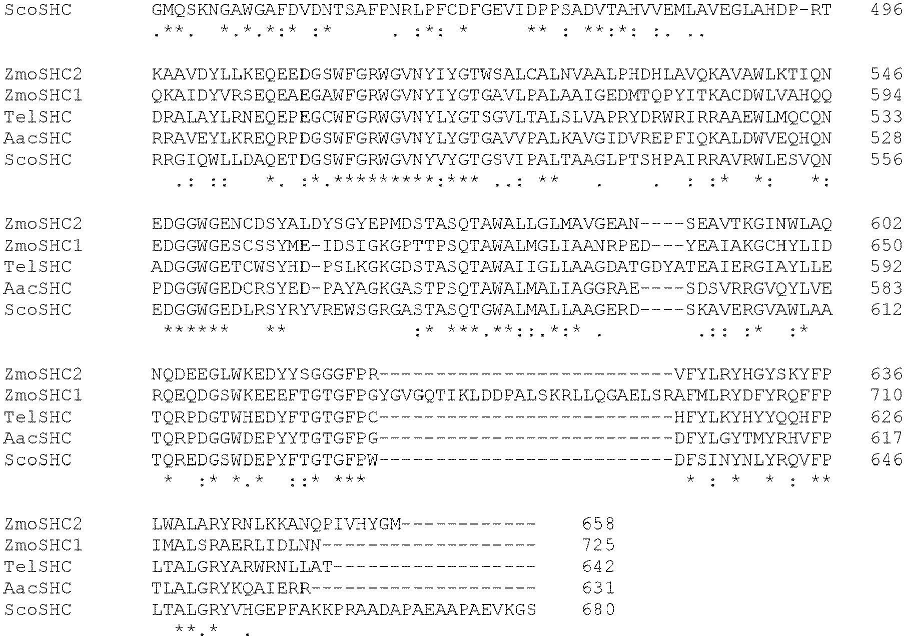

In the matrix of table 6 the various corresponding amino acid sequences are presented in rows which are aligned so that identical amino acids at corresponding positions are arranged in columns marked by * and wherein gaps in between identical and thus conserved amino acids are indicated by "-".

### References

Abd-Ella, A. et al. (2015) `The repellent DEET potentiates carbamate effects via insect muscarinic receptor interactions: An alternative strategy to control insect vector-borne diseases', PLoS ONE, 10(5), pp. 1-20.
Barbier, P. and Leser, G. (1897) `A Menthoglycol', Compt. Rend., 124, pp. 1308-1311.
Bastian, S.A. et al. (2017) `Selectivity in the Cyclization of Citronellal Introduced by Squalene Hopene Cyclase Variants', ChemCatChem, 9(23), pp. 4364-4368.
Beermann, S. et al. (2023) `Impact of climate change on vector- and rodent-borne infectious diseases.', Journal of health monitoring, 8(Suppl 3), pp. 33-61.
Borrego, L.G. et al. (2021) `Effect of the Stereoselectivity of para-Menthane-3,8-diol Isomers on Repulsion toward Aedes albopictus', Journal of Agricultural and Food Chemistry, 69(37), pp. 11095-11109.
Carroll, S.P. and Loye, J. (2006) `PMD, a registered botanical mosquito repellent with deet-like efficacy', Journal of the American Mosquito Control Association, 22(3), pp. 507-514.
Cheng, H. et al. (2009) `Cyclization of citronellal to p-menthane-3,8-diols in water and carbon dioxide', Green Chemistry, 11(8), pp. 1227-1231.
Drapeau, J. et al. (2011) 'Green synthesis of para-Menthane-3,8-diol from Eucalyptus citriodora: Application for repellent products', Comptes Rendus Chimie, 14(7-8), pp. 629-635.
Kočovský, P. *et al.* (1999) `New Lewis-acidic molybdenum(II) and tungsten(II) catalysts for intramolecular carbonyl erie and prins reactions. Reversal of the stereoselectivity of cyclization of citronellal', *Journal of Organic Chemistry,* 64(8), pp. 2765-2775.
Kurnia, I. et al. (2020) 'Synthesis of: P-menthane-3,8-diol from citronellal over lignin-derived carbon acid catalysts', New Journal of Chemistry, 44(25), pp. 10441-10447.
Stanovych, A. et al. (2023) 'Efficient and Greener Process for the Production of p-Menthane-3,8-diols Using Biosourced Ammonium Salts as Catalysts', ACS Sustainable Chemistry and Engineering, 11(16), pp. 6427-6434.
Swale, D.R. et al. (2014) `Neurotoxicity and mode of action of N, N-diethyl-Meta-toluamide (DEET)', PLoS ONE, 9(8).
World Health Organization (accessed on 10/10/2023) *Vector-borne diseases.* Available at: https://www.who.int/news-room/fact-sheets/detail/vector-borne-diseases.

## Claims

1. A process for preparing a composition comprising 1-(R)-cis-PMD, comprising the following steps:
x) providing (R)-citronellal, at least one reaction medium and at least one biocatalyst comprising or consisting of a protein with the enzymatic activity of a Squalene-hopene-cyclase (SHC), which protein comprises
- a mutated amino acid sequence relative to the wild type SHC of Alicyclobacillus acidocaldarius (wild type AacSHC) of SEQ ID No. 1, wherein the mutated amino acid sequence comprises the amino acid sequence of SEQ ID No. 1 and wherein in SEQ ID No. 1 at least the F at position 365 is replaced by an amino acid selected from the group consisting of A, C, H, I, M, P, S, T and Y or a functional equivalent of the mutated amino acid sequence or
- a mutated amino acid sequence relative to a wild type SHC (WT-SHC), wherein the mutated amino acid sequence comprises the amino acid sequence of the WT-SHC and wherein in the WT-SHC at least the F at a position in the amino acid sequence of the WT-SHC corresponding to F in the wild type AacSHC of SEQ ID No1 at position 365 is replaced by an amino acid selected from the group consisting of A, C, H, I, M, P, S, T and Y or a functional equivalent of the mutated amino acid sequence and
y) mixing the (R)-citronellal with the reaction medium and the biocatalyst so as to obtain a concentration of at least 4 mM (R)-citronellal in the reaction medium and reacting the (R)-citronellal with the biocatalyst under reaction conditions so as to convert the (R)-citronellal to a composition comprising 1-(R)-cis-PMD.

2. The process according to anyone of claim 1, wherein the reaction medium has a pH of at least 6,1, preferably 6,4 to 8,0.

3. The process according to claim 1 or 2, further comprising subsequent to step y) a step x) of isolating 1-(R)-cis-PMD from the composition comprising 1-(R)-cis-PMD obtained in step y).

4. The process according to anyone of claim 2 or 3, wherein in step x) an encapsulating agent, in particular cyclodextrin, in particular γ-cyclodextrin, is provided and mixed in step y) with the reaction medium and the biocatalyst.

5. A protein with the enzymatic activity of a Squalene-hopene-cyclase (SHC) comprising
a) a mutated amino acid sequence relative to the wild type SHC of Alicyclobacillus acidocaldarius (wild type AacSHC) of SEQ ID No. 1, wherein the mutated amino acid sequence comprises the amino acid sequence of SEQ ID No. 1 and wherein in SEQ ID No. 1 at least the F at position 365 and the L at position 607 are mutated by an amino acid replacement or a functional equivalent of the mutated amino acid sequence or
b) a mutated amino acid sequence relative to a wild type SHC (WT-SHC), wherein the mutated amino acid sequence comprises the amino acid sequence of the WT-SHC and wherein in the WT-SHC at least the F at a position in the amino acid sequence of the WT-SHC corresponding to F365 in the wild type AacSHC of SEQ ID No. 1 and the amino acid, in particular L, at a position in the amino acid sequence of the WT-SHC corresponding to L607 in the wild type AacSHC of SEQ ID No. 1 are mutated by an amino acid replacement or a functional equivalent of the mutated amino acid sequence.

6. The protein of claim 5, wherein a) in SEQ ID No. 1 at least the F at position 365 is mutated by replacement with an amino acid selected from the group consisting of A, C, H, I, M, P, S, T and Y, preferably with an amino acid selected from the group consisting of A, P, S, T and Y preferably with A, or wherein b) at least the F at a position in the amino acid sequence of the WT-SHC corresponding to F365 in the wild type AacSHC of SEQ ID No. 1 is replaced by an amino acid selected from the group consisting of A, C, H, I, M, P, S, T and Y, preferably with an amino acid selected from the group consisting of A, P, S, T and Y, preferably with A.

7. The protein of claim 5 or 6 or used in a process of claims 1 to 4, wherein further a) in SEQ ID No. 1 at least the L at position 607 is mutated by replacement with an amino acid selected from the group consisting of A, D, G, M and N, preferably with G or M, or b) at least the amino acid, in particular L, at a position in the amino acid sequence of the WT-SHC corresponding to L607 in the wild type AacSHC of SEQ ID No. 1 is replaced by an amino acid selected from the group consisting of A, D, G, M and N, preferably with G or M.

8. The protein according to anyone of claims 5 to 7 or used in a process of claims 1 to 4, wherein further a) in SEQ ID No.1 at least the G at position 600 is mutated, in particular by replacement with F, L, M or V or b) at least the G at a position in the amino acid sequence of the WT-SHC corresponding to G600 in the wild type AacSHC of SEQ ID No. 1 is mutated, in particular by replacement with F, L M or V.

9. The protein according to anyone of claims 5 to 8 or used in a process of claims 1 to 4, wherein a) the mutated amino acid sequence relative to the wild type SHC of Alicyclobacillus acidocaldarius comprises at least mutations selected from the group consisting of F365A/L607G, F365A/L697M, F365A/G600F/L607M and F365A/G600F/L607G or b) the mutated amino acid sequence relative to a wild type SHC comprises mutations selected from the group consisting of the corresponding mutations of F365A/L607G, F365A/L697M, F365A/G600F/L607M and F365A/G600F/L607G in the wild type SHC.

10. The protein according to anyone of claims 5 to 9 or used in a process of claims 1 to 4, wherein the mutated amino acid sequence relative to the wild type AacSHC of SEQ ID No. 1 is the amino acid sequence of any one of SEQ ID Nos 6 to 9, or
wherein the WT-SHC is selected from the group consisting of WT-SHC of Streptomyces coelicolor (ScoSHC, SEQ ID No. 2), Zymomonas mobilis (ZmoSHC1, SEQ ID No. 3, ZmoSHC2, SEQ ID No. 4) and Thermosynechococcus elongatus (TelSHC, SEQ ID No. 5) or wherein the mutated amino acid sequence relative to a WT-SHC of Streptomyces coelicolor, in particular of SEQ ID Nos 2, is the amino acid sequence of SEQ ID 10.

11. The protein according to any one of claims 5 to 10 or used in a process of claims 1 to 4, which comprises the set of conserved amino acids W169, D374, D376, D377, Y420, W489, Y609 and Y612 by reference to the wild type AacSHC of SEQ ID No. 1, in particular L22, Q26, G30, W32, A44, E45, L48, Q72, G76, W78, G83, A94, Y95, L98, G102, A113, I117, G121, G122, F129, T130, L134, A135, L136, G138, W142, P146, P149, I163, W169, A170, R171, P185, F217, D222, R237, G259, I261, P263, P281, S309, P310, W312, D313, T314, L316, A320, A336, W339, Q344, G349, D350, W351, P360, G361, G362, A364, F365, N369, Y372, P373, D374, D376, D377, T378, A379, A384, W406, M410, Q411, G415, W417, A419, N424, P433, D436, D442, P443, D447, V448, Q479, A450, Q479, G483, W485, F486, G487, R488, W489, G490, V491, N492, Y493, Y495, G496, T497, A503, L504, A519, W522, Q527, D530, G531, G532, W533, G534, E535, S539, Y540, T552, S554, Q555, T556, W558, A559, L563, A565, G577, L581, Q585, G589, W591, E593, G589, G600, F601, P602, F605, Y609, Y612, F616, P617, A620, L621 and R623 by reference to the wild type SHC of Alicyclobacillus acidocaldarius of SEQ ID No 1.

12. The protein according to any one of claims 5 to 11 or used in a process of claims 1 to 4, wherein the functional equivalent of the mutated amino acid sequence has an amino acid sequence identity from 38,0 to 99,9 % to the amino acid sequence of the mutated amino acid sequence.

13. A process for preparing a biocatalyst with the enzymatic activity of a SHC, comprising the following steps:
a) providing a host cell comprising a nucleic acid sequence encoding a protein according to any one of claims 5 to 12,
b) cultivating the host cell of step a) in a culture medium under conditions, which allow the expression of the protein and
c) obtaining the biocatalyst, in particular the expressed protein or a host cell comprising the expressed protein.

14. A biocatalyst, in particular prepared according to the process of claim 13, wherein the biocatalyst is the protein according to any one of claims 5 to 12, a host cell comprising the protein according to any one of claims 5 to 12, a host cell lysate, a freeze-dried host cell, a host cell extract or a host cell fraction comprising the protein according to any one of claims 5 to 12.

15. A 1-(R)-cis-PMD comprising composition obtainable by the process according to anyone of claim 1 to 4.
